# EUROPEAN PATENT APPLICATION

(11) **EP 4 837 959 A1**
(43) Date of publication of application: **11.11.2026**
(21) Application number: 24915185.3
(22) Date of filing: 27.12.2024
(51) Int. Cl.: C12N 1/21, C12P 7/22, C12P 13/22

(54) **ENGINEERED MICROORGANISM AND METHOD FOR PRODUCING AROMATIC AMINO ACIDS**

(30) Priority: 02.01.2024 WO PCT/CN2024/070048
(71) Applicant: Harbin Xiangbai Bio-Tech Co., Ltd., Harbin, Heilongjiang 150025 (CN); Beijing Institute of Technology, Haidian District Beijing 100081 (CN)
(72) Inventor: HUO, Yi-Xin, Beijing 100081 (CN); LU, Yu, Harbin, Heilongjiang 150025 (CN); CAO, Guoqiang, Harbin, Heilongjiang 150025 (CN); SUN, Lichao, Beijing 100081 (CN); LIANG, Zeyu, Beijing 100081 (CN); HUO, Runtian, Beijing 100081 (CN)
(74) Representative: Inspicos P/S
(86) International application number: PCT/CN2024/143061
(87) International publication number: WO 2025/145970

(57) **Abstract**

The present disclosure provides engineered microorganisms capable of biologically producing aromatic amino acid, wherein the engineered microorganism has reduced production of unwanted byproducts and/or increased yield or conversion rate of aromatic amino acid. Also provided are methods for preparing and using the engineered microorganism, including a method for producing aromatic amino acids.

## Description

### CROSS REFERENCE TO RELATED APPLICATIONS

The present application claims priority to PCT Patent Application PCT/CN2024/070048 filed on January 2, 2024, which are incorporated herein by reference in their entirety.

### FIELD

The present disclosure provides engineered microorganisms capable of biologically producing aromatic amino acid, having decreased production of unwanted by-products and/or increased yield or conversion rate of aromatic amino acid. Methods of making and using the same, including methods for production of aromatic amino acid are also provided.

### BACKGROUND

The aromatic amino acids, including L-tryptophan (L-TRP), L-phenylalanine (L-PHE) and L-tyrosine (L-TYR), are essential amino acids, which are utilized in a variety of applications in the food and medical industries, e.g., used as an additive in animal feed and foods and as an ingredient in many medicines. Accordingly, there is a high industrial demand for aromatic amino acids.

The aromatic amino acids are the final products of the aromatic biosynthetic pathway comprising the shikimate (SHK) pathway, which connects central carbon metabolism (CCM) with the biosynthesis of chorismate (CHA). These pathways are present in bacteria and in several eukaryotic organisms such as fungi. However, such microorganisms do not naturally overproduce these compounds in general. In order to enable production, it is necessary to functionally integrate heterologous pathways or genetically modify the naturally occurring pathways.

Given the high demand for aromatic amino acid, there remains a need in the art for alternative and improved methods for biologically producing aromatic amino acids with decreased production of unwanted by-products and/or increased efficiency of the production of aromatic amino acids.

### SUMMARY

According to a first aspect, provided is an engineered microbial cell that produces aromatic amino acid, comprising:
(a) heterologous expression of exogenous transketolase or a variant thereof, wherein the exogenous transketolase or a variant thereof possesses a higher catalytic efficiency for glyceraldehyde-3-phosphate (G3P) and/or fructose-6-phosphate (F6P) than for erythrose-4-phosphate (E4P) and/or xylulose 5-phosphate (X5P); and
(b) blocked or reduced flux through pentose phosphate pathway.

In some embodiments, the higher catalytic efficiency includes lower Km and/or higher kcat. In some embodiments, the higher catalytic efficiency at least includes lower Km.

In some embodiments, the exogenous transketolase or a variant thereof possesses a ratio of Km for G3P or F6P to Km for E4P or X5P that is less than 1:1, e.g., less than 1:1.5, 1:2, 1:2.5, 1:3, 1:3.5, 1:4, 1:4.5 or 1:5.

In some embodiments, the exogenous transketolase or a variant thereof further possesses the following characteristics: a lower Km for glyceraldehyde-3-phosphate (G3P) or sedoheptulose-7-phosphate (S7P) than for xylulose 5-phosphate (X5P) or ribose-5-phosphate (R5P); e.g., possesses a ratio of Km for G3P or S7P to Km for X5P or R5P that is less than 1:1, e.g., less than 1:1.5, 1:2, 1:2.5, 1:3, 1:3.5, 1:4, 1:4.5 or 1:5.

In some embodiments, the exogenous transketolase is derived from *Leishmania mexicana*, *Scheffersomyces stipites*, *Suhomyces tanzawaensis*, *Candida subhashii*, *Candida tenuis*, *Hyphopichia burtonii*, *Spathaspora passalidarum*, *Candida intermedia*, or *Pichia sorbitophila*.

In some embodiments, the exogenous transketolase comprises the amino acid sequence of any one of SEQ ID NOs: 1-12 or functionally equivalent variants thereof.

In some embodiments, the engineered microbial cell described above comprises blocked or reduced oxidative phase of pentose phosphate pathway. In some embodiments, the engineered microbial cell comprises reduced activity of 6-phosphogluconate dehydrogenase and/or glucose-6-phosphate dehydrogenase, e.g., achieved by deleting, knocking out or knocking down endogenous *gnd* gene or *zwf* gene, or by modifying or replacing the endogenous *gnd* gene and/or *zwf* gene to contain a rare codon.

In some embodiments, the engineered microbial cell described above further comprises enhanced flux through nitrogen assimilation pathway, e.g., comprising increased activity of glutamate dehydrogenase and/or glutamine synthetase, reduced activity of glutamate synthase, or any combination thereof. In certain embodiments, *gltBD* gene is deleted, knocked out, or knocked down. In other embodiments, *gdhA* and *glnA* genes are overexpressed. In other embodiments, *gltBD* gene is deleted, knocked out, or knocked down, and *gdhA* and *glnA* genes are overexpressed.

In some embodiments, the engineered microbial cell described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally further comprising (iii) deletion, knockout, or knockdown of endogenous *gltBD* gene.

In some embodiments, the engineered microbial cell described above further comprises reduced activity of endogenous transketolase, e.g., achieved by deleting, knocking out or knocking down endogenous *tktA* gene and/or *tktB* gene, or by modifying or replacing the endogenous *tktA* gene and/or *tktB* gene to contain a rare codon.

In some embodiments, the engineered microbial cell described above further comprises reduced activity of endogenous transaldolase, e.g., achieved by deleting, knocking out or knocking down endogenous *talA* gene and/or *talB* gene, or by modifying or replacing the endogenous *talA* gene and/or *talB* gene to contain a rare codon.

In some embodiments, at least two or more optional modifications may be combined to obtain an engineered microbial cell with desired properties. Therefore, in some embodiments, the engineered microbial cell described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene;
optionally the engineered microbial cell described above further comprises any one of the following:
(iii) deletion, knockout, or knockdown of endogenous *gltBD* gene;
(iv) deletion, knockout, or knockdown of endogenous *tktA* gene and/or *tktB* gene;
(v) deletion, knockout, or knockdown of endogenous *talA* gene and/or *talB* gene;
(vi) any combination of (iii) to (v).

In some embodiments, any of the aforementioned engineered microbial cell described herein optionally further comprises one or more additional genetic modifications that facilitate or optimize the biosynthesis of aromatic amino acids.

In some embodiments, the engineered microbial cell described above optionally further comprises blocked or attenuated flux through TCA cycle. In some embodiments, the engineered microbial cell comprises reduced activity of pyruvate dehydrogenase and/or citrate synthase, e.g., achieved by deleting, knocking out or knocking down *lpd*, *aceF*, *aceE* gene or *gltA* gene, or by modifying or replacing the endogenous *lpd*, *aceF*, *aceE* gene or *gltA* gene to contain a rare codon. In some embodiments, the engineered microbial cell described comprises reduced activity of citrate synthase, e.g., achieved by modifying or replacing the endogenous *gltA* gene to contain a rare codon.

In some embodiments, the engineered microbial cell described above further comprises blocked or reduced oxaloacetate biosynthesis pathway. In some embodiments, the engineered microbial cell comprises reduced activity of phosphoenolpyruvate carboxylase, e.g., achieved by deleting, knocking out or knocking down *ppc*, or by modifying or replacing the endogenous *ppc* gene to contain a rare codon.

In some embodiments, the engineered microbial cell described above further comprises the naturally occurring phosphotransferase (PTS) transport system that is disabled, blocked or attenuated. In some embodiments, the engineered microbial cell comprises reduced activity of one or more PTS system protein (s), e.g., achieved by deleting, knocking out or knocking down one or more gene(s) selected from *ptsH*, *ptsI*, *crr*, *ptsG*, or any combination thereof, or by modifying or replacing the endogenous *ptsH*, *ptsI*, *crr*, *ptsG* gene, or any combination thereof to contain a rare codon.

In some embodiments, the engineered microbial cell further comprises increased activity of at least one non-PTS sugar transporter, e.g., overexpression of endogenous non-PTS sugar transporter or heterologous expression of exogenous non-PTS sugar transporter, such as glucose facilitator (Glf), e.g., from *Zymomonas mobilis*. In some embodiments, the engineered microbial cell further comprises increased activity of glucokinase, e.g., overexpression of endogenous *glk* gene or heterologous expression of exogenous *glk* gene, e.g., from *Zymomonas mobilis*. In some embodiments, the engineered microbial cell comprises: deletion, knockout, or knockdown of one or more gene(s) selected from *ptsH*, *ptsI*, *crr*, *ptsG*, or any combination thereof, or modification or replacement of endogenous *ptsH*, *ptsI*, *crr*, *ptsG*, or any combination thereof to contain a rare codon; and overexpression of endogenous *glf* gene or heterologous expression of exogenous *glk* gene, e.g., from *Zymomonas mobilis*.

In some embodiments, the engineered microbial cell described above further comprises blocked or reduced pathways for production of acetic acid and/or lactic acid. In some embodiments, the engineered microbial cell comprises reduced activity of one or more enzymes selected from phosphate acetyltransferase, pyruvate dehydrogenase, lactate dehydrogenase, or any combination thereof; e.g., achieved by deleting, knocking out or knocking down one or more gene(s) selected from *pta*, *poxB*, *ldhA*, or any combination thereof, or by modifying or replacing the endogenous *pta*, *poxB*, *ldhA* gene, or any combination thereof to contain a rare codon.

In some embodiments, the engineered microbial cell described above further comprises an enhanced phosphoenolpyruvate (PEP) biosynthesis pathway. In certain embodiments, the engineered microbial cell comprises increased activity of phosphoenolpyruvate synthase to enhance conversion from pyruvate to PEP, e.g., achieved by overexpression of endogenous *ppsA* or heterologous expression of exogenous *ppsA* gene. In other embodiments, the engineered microbial cell comprises increased activity of pyruvate carboxylase and PEP carboxykinase to enhance conversion from pyruvic acid to PEP, e.g., achieved by combined overexpression of both endogenous or exogenous *pyc* gene and endogenous or exogenous *pck* genes, e.g., achieved by overexpression of endogenous *pyc* and *pck* genes, or heterologous expression of exogenous *pyc* and *pck* genes, or achieved by overexpression of endogenous *pck* gene and heterologous expression of exogenous *pyc* gene (e.g., from *Cytobacillus firmus*).

In some embodiments, at least two or more optional modifications may be combined to obtain an engineered microbial cell with desired properties. Therefore, in some embodiments, the engineered microbial cell described above comprises:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and
(ii) deletion, knockout, or knockdown of endogenous *gnd* gene and/or *zwf* gene;
optionally the engineered microbial cell described above further comprises any one of the following:
(iii) deletion, knockout, or knockdown of endogenous *gltBD* gene;
(iv) deletion, knockout, or knockdown of endogenous *tktA* gene and/or *tktB* gene;
(v) deletion, knockout, or knockdown of endogenous *talA* gene and/or *talB* gene;
(vi) modification or replacement of endogenous *gltA* gene to contain a rare codon;
(vii) deletion, knockout, or knockdown of endogenous *ppc* gene;
(viii) deletion, knockout, or knockdown of endogenous *ptsH*, *ptsI*, *crr*, *ptsG* gene, or any combination thereof;
(ix) overexpression of endogenous *glf* gene or *glk* gene or heterologous expression of exogenous *glf* gene or *glk* gene;
(x) deletion, knockout, or knockdown of endogenous *pta*, *poxB*, *ldhA* gene, or any combination thereof;
(xi) overexpression of endogenous *ppsA*, *pyc*, *pck* gene or any combination thereof; or heterologous expression of exogenous *ppsA*, *pyc*, *pck* gene or any combination thereof;
(xii) any combination of (iii) to (xi).

Any of the aforementioned engineered microbial cell described herein may be bacteria, fungi, or yeast. In some embodiments, the engineered microbial cell is *Escherichia coli, Corynebacterium glutamicum*, *Bacillus subtilis*, or *Bacillus licheniformi*.

According to a second aspect, also provided is use of any of the aforementioned engineered microbial cell described herein in biosynthesis of aromatic amino acid.

According to a third aspect, also provided is method for producing aromatic amino acid, comprising culturing any of the aforementioned engineered microbial cell described herein in a medium. In some embodiments, the method further comprises recovering aromatic amino acid from the culture.

According to a fourth aspect, also provided is a method for decreasing the production of by-products and/or increasing the yield or conversion rate of aromatic amino acid in aromatic amino acid biosynthesis, comprising introducing a genetic modification as defined in the first aspect to a microbial cell that possesses an aromatic amino acid biosynthesis pathway.

Further features and advantages of certain embodiments of the present disclosure will become more fully apparent in the following description of the embodiments and drawings thereof, and from the claims.

### BRIEF DESCRIPTION OF THE DRAWINGS

Figure 1. Knockout of *gnd* gene affects the growth of the strains.
Figure 2. Introduction of exogenous *tkt* gene reduces the accumulation of byproducts.
Figure 3. Introduction of exogenous *tkt* mutant increases tryptophan production.
Figure 4. Introduction of exogenous *tkt* mutant and overexpression of *ppsA* increases tryptophan production.
Figure 5. Knockout of *gltBD* increases tryptophan production.
Figure 6. Modification of nitrogen cycle increases tryptophan conversion rate.
Figure 7. Combined modifications of the nitrogen cycle and *tkt* strategy enhance tryptophan production.
Figure 8. Combined modifications of the carbon cycle, nitrogen cycle, and *tkt* strategy increase the tryptophan conversion rate.
Figure 9. Combined modifications of the *tkt* strategy and TCA attenuation strategy increase the tryptophan conversion rate.
Figure 10. Knockout of *zwf* increases tryptophan conversion rate.

### DETAILED DESCRIPTION

### Definitions

The genes disclosed in the present disclosure are all known in the art and their sequences are available from public databases. The following exemplary sequences for the genes and gene products are provided.

| Gene | NCBI GeneID | Gene product | NCBI Reference Sequence: |
|---|---|---|---|
| *tktA* | 947420 | transketolase 1 | YP_026188.1 |
| *tktB* | 945865 | transketolase 2 | NP_416960.1 |
| *gnd* | 946554 | 6-phosphogluconate dehydrogenase | NP_416533.1 |
| *zwf* | 946370 | NADP(+)-dependent glucose-6-phosphate dehydrogenase | NP_416366.1 |
| *gltB* | 947724 | glutamate synthase subunit GltB | NP_417679.2 |
| *gltD* | 947723 | glutamate synthase subunit GltD | NP_417680.1 |
| *gdhA* | 946802 | glutamate dehydrogenase | NP_416275.1 |
| *glnA* | 948370 | glutamine synthetase | NP_418306.1 |
| *gltA* | 945323 | citrate synthase | NP_415248.1 |
| *trpA* | 946204 | tryptophan synthase subunit alpha | NP_415776.1 |
| *trpB* | 945768 | tryptophan synthase subunit beta | NP_415777.1 |
| *trpC* | 945519 | fused indole-3-glycerol phosphate synthase/phosphoribosylanthranilate isomerase | NP_415778.3 |
| *trpD* | 945109 | anthranilate synthase subunit TrpD | NP_415779.1 |
| *trpE* | 945846 | anthranilate synthase subunit TrpE | NP_415780.1 |
| trpE(fbr) | / | anthranilate synthase subunit TrpE (A63V) | DOI:10.1007/s00449-021-02552-4 |
| *ptsH* | 946886 | phosphocarrier protein HPr | NP_416910.1 |
| *ptsI* | 946879 | PTS enzyme I | NP_416911.1 |
| *ptsG* | 945651 | glucose-specific PTS enzyme IIBC component | NP_415619.1 |
| *crr* | 946880 | soluble glucose-specific enzyme IIA^{Glc} (PTS system glucose-specific EIIA component) | NP_416912.1 |
| *glf* | / | UDP-galactopyranose mutase [Zymomonas mobilis] | WP_014500577.1 |
| *glk* | 79904427 | glucokinase [Zymomonas mobilis] | WP_011240290.1 |
| *pta* | 946778 | phosphate acetyltransferase | NP_416800.1 |
| *poxB* | 946132 | pyruvate oxidase | NP_415392.1 |
| *ldhA* | 946315 | D-lactate dehydrogenase | NP_415898.1 |
| *ppsA* | 946209 | phosphoenolpyruvate synthetase | NP_416217.1 |
| *pyc* | 67523489 | pyruvate carboxylase [Cytobacillus firmus] | WP_061793417.1 |
| *pck* | 945667 | phosphoenolpyruvate carboxykinase (ATP) | NP_417862.1 |
| *aroG* | 945605 | 3-deoxy-7-phosphoheptulonate synthase | NP_415275.1 |
| aroG (fbr) | / | 3-deoxy-7-phosphoheptulonate synthase (S211F) | DOI:10.1007/s00449-021-02552-4 |
| *serA* | 945258 | phosphoglycerate dehydrogenase | NP_417388.1 |
| *pheA* | 947081 | fused chorismate mutase/prephenate dehydratase | NP_417090.1 |
| *tyrB* | 948563 | tyrosine aminotransferase | NP_418478.1 |
| *tyrA* | 947115 | fused chorismate mutase/prephenate dehydrogenase | NP_417091.1 |
| *tyrB* | 948563 | tyrosine aminotransferase | NP_418478.1 |
| *talA* | 947006 | transaldolase A | NP_416959.1 |
| *talB* | 944748 | transaldolase B | NP_414549.1 |
| *lpd* | 944854 | lipoamide dehydrogenase | NP_414658.1 |
| *aceF* | 944794 | pyruvate dehydrogenase, E2 subunit | NP_414657.1 |
| *aceE* | 944834 | pyruvate dehydrogenase E1 component | NP_414656.1 |

The abbreviations used herein have their conventional meaning within the chemical and biological arts. Some abbreviations used herein: aromatic amino acid (AAA), tryptophan (TRP), phenylalanine (PHE), tyrosine (TYR), phosphoenolpyruvate (PEP), chorismate (CHA), pyruvate (PYR), glyceraldehyde-3-phosphate (G3P), fructose-6-phosphate (F6P), erythrose-4-phosphate (E4P), xylulose 5-phosphate (X5P), sedoheptulose-7-phosphate (S7P), ribose-5-phosphate (R5P), pentose phosphate pathway (HMP), tricarboxylic acid cycle (TCA), phosphoenolpyruvate: sugar phosphotransferase system (PTS).

In the present disclosure, any concentration range, percentage range, ratio range, or integer range is to be understood to include the value of any integer within the recited range and, when appropriate, fractions thereof, unless otherwise indicated. As used herein, the term "about" means ±10% (e.g., ±5%, ±2%, or ±1%) of the indicated range or value, unless otherwise indicated. It should be understood that the terms "a" and "an" as used herein refer to "one or more" of the enumerated components. The use of the alternative (e.g., "or") should be understood to mean either one, both, or any combination thereof of the alternatives.

In the present disclosure, the scientific and technical terms used herein have the meanings commonly understood by those skilled in the art, unless otherwise stated. For better understanding of the present disclosure, definitions and explanations of terms are provided below.

As used herein, the term "aromatic amino acid(s)" refers to tryptophan (TRP), phenylalanine (PHE), and tyrosine (TYR). In general, amino acids biosynthesized by organisms are typically in L-configuration. Therefore, in some embodiments of the present invention, tryptophan, phenylalanine, and tyrosine are of L-configuration. In the examples herein, tryptophan is used as an example to illustrate the advantages of the engineered microbial cells of the present invention. However, it is readily understood that the engineered microbial cells of the present invention may also be utilized for producing other aromatic amino acids, such as phenylalanine (PHE) and tyrosine (TYR).

As used herein, the term "Km", also known as Michaelis constant, refers to the substrate concentration at which the reaction rate is 50% of the Vmax (the maximum rate of the reaction when all the enzyme's active sites are saturated with substrate). Km is a measure of the affinity an enzyme has for its substrate, as the lower the value of Km, the more efficient the enzyme is at carrying out its function at a lower substrate concentration.

The term "kcat" is the turnover number that describes the number of times each enzyme site converts substrate to product per unit time. The higher the kcat is, the more substrates get turned over in one second.

In certain embodiments, Km and kcat for transketolase can be measured as follows: in the forward reaction, the kinetic parameters of transketolase were determined using auxiliary enzymes triosephosphate isomerase and glycerol-3-phosphate dehydrogenase to detect D-glyceraldehyde-3-phosphate converted from D-xylulose-5-phosphate and its pairing aldehyde recipient (Sprenger GA, et al. Eur J Biochem. 1995;230(2):525-532.); in the backward reaction, the kinetic parameters of transketolase were determined using sorbitol dehydrogenase to detect D-xylulose-5-phosphate converted from D-sedoheptulose-7-phosphate and its pairing aldehyde recipient. The transketolase-catalyzed reactions were performed at 25°C in a 100 µl reaction solution containing 20 mM Tris at pH 8.0, 100 mM NaCl, 1 mM ThDP, 0.1 mM NADH, 1 U ml⁻¹ glycerol-3-phosphate dehydrogenase and triosephosphate isomerase for the forward reactions, or 1 U ml⁻¹ sorbitol dehydrogenase for the backward reactions. The consumption of NADH was monitored continuously at 340 nm for 10 min using a Beckman DU-800 spectrophotometer (Beckmen Coulter, Fullertron, CA, USA).

As used herein, the term "endogenous" refers to a nucleic acid or protein that is present or expressed in the wild-type or parental microorganism from which the engineered microorganism disclosed herein is derived. For example, an endogenous gene is a gene that is natively present in the wild-type or parental microorganism from which the engineered microorganism disclosed herein is derived. In one embodiment, the expression of an endogenous gene may be controlled by an exogenous regulatory element, such as an exogenous promoter.

As used herein, the term "exogenous" refers to a nucleic acid or protein that originates outside the engineered microorganism disclosed herein. For example, an exogenous gene or enzyme may be artificially or recombinantly created and introduced to or expressed in the engineered microorganism disclosed herein. An exogenous gene or enzyme may also be isolated from a heterologous microorganism and introduced to or expressed in the engineered microorganism disclosed herein. Exogenous nucleic acids may be adapted to integrate into the genome of the engineered microorganism disclosed herein or to remain in an extra-chromosomal state in the engineered microorganism disclosed herein, for example, in a plasmid.

As used herein, the term "heterologous" refers to a nucleic acid or protein that is not present in the wild-type or parental microorganism from which the engineered microorganism disclosed herein is derived. For example, a heterologous gene or enzyme may be derived from a different strain or species and introduced to or expressed in the engineered microorganism disclosed herein. The heterologous gene or enzyme may be introduced to or expressed in the engineered microorganism disclosed herein in the form in which it occurs in the different strain or species. Alternatively, the heterologous gene or enzyme may be modified in some way, e.g., by codon-optimizing it for expression in the engineered microorganism disclosed herein or by engineering it to alter function, such as to reverse the direction of enzyme activity or to alter substrate specificity.

As used herein, the term "heterologous expression" involves the introduction of gene encoding for a protein of interest from one species or cell into another species or cell, thus allowing for the host cells to express the foreign protein.

As used herein, the terms "microbe", "microorganism", or "microbial cell" are intended to mean any organism that exists as a microscopic cell that is included within the domains of archaea, bacteria or eukarya. Therefore, the term is intended to encompass prokaryotic or eukaryotic cells or organisms having a microscopic size and includes bacteria, archaea and eubacteria of all species as well as eukaryotic microorganisms such as yeast and fungi.

As used herein, the term "host" refers to a cell or microorganism that may be genetically modified, e.g., by mutation, with an exogenous nucleic acid molecule, by knockout or combinations thereof, to improve the production of aromatic amino acids over the unmodified host cell. In certain embodiments, a host cell may optionally already possess other genetic modifications that confer other desired properties.

As used herein, the term "parent cell" refers to a cell that has an identical genetic background as a host cell disclosed herein except that it does not comprise a particular genetic modification (e.g., mutation or knockout of endogenous nucleic acid, introduction of exogenous or heterologous nucleic acid, or combinations thereof), and that serves as the starting point for introducing said genetic modification leading to the generation of a host cell disclosed herein. Furthermore, it is readily understood that when describing relative changes (e.g., blocking, reducing, attenuating, knocking down, enhancing, elevating, increasing, etc.) of a substance such as a gene, protein, or pathway, if a reference object is not specified, the reference object may be the corresponding substance, such as the gene, protein, or pathway, in a wild-type cell or a parental cell.

As used herein, the term "overexpression" refers to a level of gene expression or gene product in a non-natural or recombinant microorganism that is greater than the level of gene expression or gene product found in a parent or wild-type microorganism when grown under the same conditions. In certain embodiments, overexpression may occur at the transcriptional level, translational level, or both, which may be due to altered regulatory control (e.g., use of a strong promoter) or an increase in copy number or both.

As used herein, the terms "deletion", "knockout", "knockdown" have meanings generally understood by those skilled in the art. The term "deletion" refers to the removal or disruption of a specific gene within an organism's genome, preventing its normal expression, transcription, or translation. The term "knockout" refers to the complete removal or inactivation of the DNA sequence of a target gene from a cell or organism, blocking its transcription and translation processes, thereby causing the target gene to lose its function entirely. Deletion or knockout of an endogenous gene can be achieved by any known gene-editing system in the art, such as zinc finger nucleases (ZFN), transcription activator-like effector nucleases (TALEN), or clustered regularly interspaced short palindromic repeats (CRISPR). The term "knockdown" refers to reducing the expression level of a target gene in a cell or organism through gene silencing, resulting in transient alterations in expression. Exemplary gene-silencing methods include, but are not limited to, RNA interference (RNAi), small interfering RNA (siRNA), or short hairpin RNA (shRNA), which are used to inactivate the messenger RNA of a specific gene and effectively inhibit its expression.

As used herein, the term "rare codon(s)" refers to a codon encoding an amino acid that is used with lower frequency during gene expression in a particular species or cell. For example, when expressing a foreign gene in *Escherichia coli*, a rare codon that are infrequently used by *E. coli* reduce the availability of homologous tRNAs, thereby decreasing the efficiency and level of protein expression. For a specific species or cell, those skilled in the art can readily identify a rare codon using various analytical tools, such as bioinformatics tools.

As used herein, the term "vector" refers to a nucleic acid vehicle which can have a polynucleotide inserted therein. When the vector allows for the expression of the protein encoded by the polynucleotide inserted therein, the vector is called an expression vector. The vector can have the carried genetic material elements expressed in a host cell by transformation, transduction, or transfection into the host cell. Vectors are well known by a person skilled in the art, including, but not limited to plasmids, phages, cosmids, artificial chromosome such as yeast artificial chromosome (YAC), bacterial artificial chromosome (BAC) or P1-derived artificial chromosome (PAC); phage such as λ phage or M13 phage and animal virus. The animal viruses that can be used as vectors, include, but are not limited to, retrovirus (including lentivirus), adenovirus, adeno-associated virus, herpes virus (such as herpes simplex virus), pox virus, baculovirus, papillomavirus, papova virus (such as SV40). A vector may comprise multiple elements for controlling expression, including, but not limited to, a promoter sequence, a transcription initiation sequence, an enhancer sequence, a selection element, and a reporter gene. In addition, a vector may comprise origin of replication.

As used herein, the term "homolog" describes those nucleotide sequences that have sequence similarity and encode polypeptides that share at least one functional characteristic such as a biochemical activity, or those polypeptides that have sequence similarity and at the same time have at least one functional similarity such as a biochemical activity.

As used herein, the term "ortholog" refers to a homologous gene or protein that is the functional equivalent of the referenced gene or protein in another species. Orthologous sequences are homologous sequences in different species that originate by vertical descent from a single sequence of the last common ancestor, wherein the sequence and its main function are conserved. A homologous sequence is a sequence inherited in two species by a common ancestor. The term "ortholog" when used in reference to an amino acid or nucleotide/nucleic acid sequence from a given species refers to the same amino acid or nucleotide/nucleic acid sequence from a different species. It should be understood that two sequences are orthologs of each other when they are derived from a common ancestor sequence via linear descent and/or are otherwise closely related in terms of both their sequence and their biological function. Orthologs will usually have a high degree of sequence identity but may not (and often will not) share 100% sequence identity.

As used herein, the term "paralog" describes homologous sequences that originate by a sequence duplication event. Paralogous sequences often belong to the same species, but this is not necessary. Paralogs can be split into in-paralogs (paralogous pairs that arose after a speciation event) and out-paralogs (paralogous pairs that arose before a speciation event). Between species out-paralogs are pairs of paralogs that exist between two organisms due to duplication before speciation. Within species out-paralogs are pairs of paralogs that exist in the same organism, but whose duplication event happened after speciation. Paralogs typically have the same or similar function.

As used herein, the term "variant" is a polynucleotide or polypeptide that differs from a reference polynucleotide or polypeptide respectively but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A variant of a polynucleotide or polypeptide may be naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to the persons skilled in the art. Due to the retention of fundamental properties (or functional characteristics), variant is also referred to herein as functional variant or functionally equivalent variant(s).

As used herein, the term "identity" refers to a measure of similarity between nucleotide sequences or amino acid sequences. Generally, sequences were aligned to obtain a maximum matching. "Identity" has well-known meanings in the art and can be calculated by published algorithm (such as BLAST).

### Engineered microbes producing AAAs

This disclosure provides engineered biosynthetic routes to facilitate or optimize the biosynthesis of aromatic amino acids.

In accordance with some embodiments, the present disclosure increases the production efficiency of aromatic amino acids at least by (i) introducing exogenous transketolase with specific substrates selectivity to generate erythrose-4-phosphate (E4P) independent of pentose phosphate pathway; and (ii) reducing carbon flux into oxidative phase of pentose phosphate pathway.

In certain aspects, provided herein is an engineered microbial cell that produces aromatic amino acid, comprising:
(a) heterologous expression of exogenous transketolase, wherein the exogenous transketolase or a variant thereof possesses a higher catalytic efficiency or transketolase activity for glyceraldehyde-3-phosphate (G3P) and/or fructose-6-phosphate (F6P) than for erythrose-4-phosphate (E4P) and/or xylulose 5-phosphate (X5P); and
(b) blocked or reduced flux through pentose phosphate pathway.

### Transketolase

Introduction of the exogenous transketolase or its variant as provided herein controls the reaction directionality from G3P + F6P to E4P +X5P independent of pentose phosphate pathway. Reaction directionality can be controlled, e.g., by providing a transketolase possessing a higher catalytic efficiency for G3P and/or F6P than for E4P and/or X5P; or by providing a transketolase possessing a higher catalytic activity (i.e., transketolase activity) for G3P and/or F6P than for E4P and/or X5P.

In some embodiments, the exogenous transketolase or its variant as provided herein also controls the reaction directionality from G3P + S7P to X5P + R5P independent of pentose phosphate pathway. Reaction directionality can be controlled, e.g., by providing a transketolase possessing a higher catalytic efficiency for G3P and/or S7P than for X5P and/or R5P; or by providing a transketolase possessing a higher catalytic activity (i.e., transketolase activity) for G3P and/or S7P than for X5P and/or R5P.

In some embodiments, the exogenous transketolase or its variant at least possesses a higher catalytic efficiency or transketolase activity for fructose-6-phosphate (F6P) than for xylulose 5-phosphate (X5P), and optionally further possesses a higher catalytic efficiency or transketolase activity for sedoheptulose-7-phosphate (S7P) than for xylulose 5-phosphate (X5P).

### I. Catalytic activity

Transketolase catalyzes the reversible transfer of two-carbon (1,2-dihydroxyethyl) units from ketose phosphates to the C1 position of aldose phosphates and thus provides, together with the Schiff base-forming transaldolase, a reversible link between glycolysis and the pentose phosphate pathway. Transketolase acts on different ketose phosphate (donor) and aldose phosphate (acceptor) substrates of variable carbon chain length (3-7 carbons) in two major, essentially reversible reactions: $R 5 P + X 5 P ⇔ S 7 P + G 3 P ;$ $E 4 P + X 5 P ⇔ G 3 P + F 6 P .$

In some embodiments, the exogenous transketolase or its variant possesses a higher transketolase activity for glyceraldehyde-3-phosphate (G3P) and/or fructose-6-phosphate (F6P) than for erythrose-4-phosphate (E4P) and/or xylulose 5-phosphate (X5P).

In some embodiments, the exogenous transketolase or its variant possesses one or more of: (i) a higher transketolase activity for glyceraldehyde-3-phosphate (G3P) than for erythrose-4-phosphate (E4P); (ii) a higher transketolase activity for glyceraldehyde-3-phosphate (G3P) than for xylulose 5-phosphate (X5P); (iii) a higher transketolase activity for fructose-6-phosphate (F6P) than for erythrose-4-phosphate (E4P); (iv) a higher transketolase activity for fructose-6-phosphate (F6P) than for xylulose 5-phosphate (X5P); or any combination thereof.

In some embodiments, the exogenous transketolase and the variant at least possesses a higher transketolase activity for fructose-6-phosphate (F6P) than for xylulose 5-phosphate (X5P).

In some embodiments, the exogenous transketolase and the variant possesses a ratio of transketolase activity for G3P or F6P to transketolase activity for E4P or X5P that is higher than 1:1. In some embodiments, the ratio is higher than 1.5:1, 2:1, 2.5:1, 3:1, 3.5:1, 4:1, 4.5:1 or 5:1.

In some embodiments, the exogenous transketolase and the variant possesses one or more of: (i) a ratio of transketolase activity for G3P to transketolase activity for E4P that is higher than 1:1, e.g., higher than 1.5:1, 2:1, 2.5:1, 3:1, 3.5:1, 4:1, 4.5:1 or 5:1; (ii) a ratio of transketolase activity for G3P to transketolase activity for X5P that is higher than 1:1, e.g., higher than 1.5:1, 2:1, 2.5:1, 3:1, 3.5:1, 4:1, 4.5:1 or 5:1; (iii) a ratio of transketolase activity for F6P to transketolase activity for E4P that is higher than 1:1, e.g., higher than 1.5:1, 2:1, 2.5:1, 3:1, 3.5:1, 4:1, 4.5:1 or 5:1; (iv) a ratio of transketolase activity for F6P to transketolase activity for X5P that is higher than 1:1, e.g., higher than 1.5:1, 2:1, 2.5:1, 3:1, 3.5:1, 4:1, 4.5:1 or 5:1; or any combination thereof.

In some embodiments, the exogenous transketolase and the variant at least possesses a ratio of transketolase activity for F6P to transketolase activity for X5P that is higher than 1:1, e.g., higher than 1:1.5, 1:2, 1:2.5, 1:3, 1:3.5, 1:4, 1:4.5 or 1:5, e.g., higher than 1:4.

In some embodiments, the exogenous transketolase and the variant as described herein further possesses a higher transketolase activity for glyceraldehyde-3-phosphate (G3P) or sedoheptulose-7-phosphate (S7P) than for xylulose 5-phosphate (X5P) or ribose-5-phosphate (R5P). In some embodiments, the exogenous transketolase and the variant possesses one or more of: (a) a higher transketolase activity for glyceraldehyde-3-phosphate (G3P) than for xylulose 5-phosphate (X5P); (b) a higher transketolase activity for glyceraldehyde-3-phosphate (G3P) than for ribose-5-phosphate (R5P); (c) a higher transketolase activity for sedoheptulose-7-phosphate (S7P) than for xylulose 5-phosphate (X5P); (d) a higher transketolase activity for sedoheptulose-7-phosphate (S7P) than for ribose-5-phosphate (R5P); or any combination thereof.

In some embodiments, the exogenous transketolase and the variant possesses a ratio of transketolase activity for G3P or S7P to transketolase activity for X5P or R5P that is higher than 1:1. In some embodiments, the ratio is higher than 1.5:1, 2:1, 2.5:1, 3:1, 3.5:1, 4:1, 4.5:1 or 5:1.

In some embodiments, the exogenous transketolase and the variant possesses one or more of: (a) a ratio of transketolase activity for G3P to transketolase activity for X5P that is higher than 1:1, e.g., higher than 1.5:1, 2:1, 2.5:1, 3:1, 3.5:1, 4:1, 4.5:1 or 5:1; (b) a ratio of transketolase activity for G3P to transketolase activity for R5P that is higher than 1:1, e.g., higher than 1.5:1, 2:1, 2.5:1, 3:1, 3.5:1, 4:1, 4.5:1 or 5:1; (c) a ratio of transketolase activity for S7P to transketolase activity for X5P that is higher than 1:1, e.g., higher than 1.5:1, 2:1, 2.5:1, 3:1, 3.5:1, 4:1, 4.5:1 or 5:1; (d) a ratio of transketolase activity for S7P to transketolase activity for R5P that is higher than 1:1, e.g., higher than 1.5:1, 2:1, 2.5:1, 3:1, 3.5:1, 4:1, 4.5:1 or 5:1; or any combination thereof.

In some embodiments, the exogenous transketolase or its variant possesses a ratio of transketolase activity for S7P to transketolase activity for X5P that is higher than 1:1, e.g., higher than 1.5:1, 2:1, 2.5:1, 3:1, 3.5:1, 4:1, 4.5:1 or 5:1, e.g., higher than 4:1.

In some embodiments, the exogenous transketolase and the variant at least possesses a higher transketolase activity for fructose-6-phosphate (F6P) than for xylulose 5-phosphate (X5P), and optionally further possesses a higher transketolase activity for sedoheptulose-7-phosphate (S7P) than for xylulose 5-phosphate (X5P).

In some embodiments, the exogenous transketolase or its variant possesses a ratio of transketolase activity for F6P to transketolase activity for X5P that is higher than 1:1 (e.g., 4:1) and a ratio of transketolase activity for S7P to transketolase activity for X5P that is higher than 1:1 (e.g., 4:1).

### II. Catalytic efficiency

In some embodiments, the exogenous transketolase or its variant possesses a higher catalytic efficiency for glyceraldehyde-3-phosphate (G3P) and/or fructose-6-phosphate (F6P) than for erythrose-4-phosphate (E4P) and/or xylulose 5-phosphate (X5P).

The term "catalytic efficiency" as described herein, can be given by any enzyme kinetic parameters known to those skilled in the art, such as Km, kcat, and the ratio kcat/Km.

The value of Km is inversely related to the affinity and the catalytic efficiency of the enzyme for its substrate. High values of Km correspond to low affinity toward substrate and thus low catalytic efficiency for substrate. Low Km values for an enzyme correspond to high affinity toward substrate and thus high catalytic efficiency for substrate.

The value of kcat is positively related to the catalytic efficiency of the enzyme for its substrate. High value of kcat correspond to high catalytic efficiency for substrate. Low kcat value for an enzyme correspond to low catalytic efficiency for substrate.

In some embodiments, the higher catalytic efficiency as described herein includes lower Km and/or higher kcat. In some embodiments, the higher catalytic efficiency as described herein at least includes lower Km.

### II-A. Km value

In some embodiments, the engineered microbial cell as described herein comprises an exogenous transketolase or a variant thereof possessing one or more of: (i) a lower Km for glyceraldehyde-3-phosphate (G3P) than for erythrose-4-phosphate (E4P); (ii) a lower Km for glyceraldehyde-3-phosphate (G3P) than for xylulose 5-phosphate (X5P); (iii) a lower Km for fructose-6-phosphate (F6P) than for erythrose-4-phosphate (E4P); (iv) a lower Km for fructose-6-phosphate (F6P) than for xylulose 5-phosphate (X5P); or any combination thereof. Introduction of exogenous transketolase or a variant thereof with specific substrates selectivity, controls the reaction directionality from G3P + F6P to E4P +X5P independent of pentose phosphate pathway. In some embodiments, the variant may share high structural similarity with the wild-type transketolase from which it is derived. In some embodiments, the variant may have at least 80% (e.g., at least 85%, at least 90%, at least 91%, at least 92%, at least 92%, at least 93%, at least 94%, at least 95%, at least 96%, at least 97%, at least 98%, or at least 99%) sequence identity compared to the wild-type transketolase from which it is derived. In some embodiments, the variant may have one or more substitutions (e.g., one substitution) compared to the wild-type transketolase from which it is derived. The variant retains the specific substrates selectivity of the wild-type transketolase.

In some embodiments, the exogenous transketolase and the variant at least possesses a lower Km for fructose-6-phosphate (F6P) than for xylulose 5-phosphate (X5P).

In some embodiments, the exogenous transketolase and the variant possesses a ratio of Km for G3P or F6P to Km for E4P or X5P that is less than 1:1. In some embodiments, the ratio is less than 1:1.5, 1:2, 1:2.5, 1:3, 1:3.5, 1:4, 1:4.5 or 1:5.

In some embodiments, the exogenous transketolase and the variant possesses one or more of: (i) a ratio of Km for G3P to Km for E4P that is less than 1:1, e.g., less than 1:1.5, 1:2, 1:2.5, 1:3, 1:3.5, 1:4, 1:4.5 or 1:5; (ii) a ratio of Km for G3P to Km for X5P that is less than 1:1, e.g., less than 1:1.5, 1:2, 1:2.5, 1:3, 1:3.5, 1:4, 1:4.5 or 1:5; (iii) a ratio of Km for F6P to Km for E4P that is less than 1:1, e.g., less than 1:1.5, 1:2, 1:2.5, 1:3, 1:3.5, 1:4, 1:4.5 or 1:5; (iv) a ratio of Km for F6P to Km for X5P that is less than 1:1, e.g., less than 1:1.5, 1:2, 1:2.5, 1:3, 1:3.5, 1:4, 1:4.5 or 1:5; or any combination thereof.

In some embodiments, the exogenous transketolase and the variant at least possesses a ratio of Km for F6P to Km for X5P that is less than 1:1, e.g., less than 1:1.5, 1:2, 1:2.5, 1:3, 1:3.5, 1:4, 1:4.5 or 1:5, e.g., less than 1:4.

In some embodiments, the exogenous transketolase and the variant as described herein further possesses a lower Km for glyceraldehyde-3-phosphate (G3P) or sedoheptulose-7-phosphate (S7P) than for xylulose 5-phosphate (X5P) or ribose-5-phosphate (R5P). In some embodiments, the exogenous transketolase and the variant possesses one or more of: (a) a lower Km for glyceraldehyde-3-phosphate (G3P) than for xylulose 5-phosphate (X5P); (b) a lower Km for glyceraldehyde-3-phosphate (G3P) than for ribose-5-phosphate (R5P); (c) a lower Km for sedoheptulose-7-phosphate (S7P) than for xylulose 5-phosphate (X5P); (d) a lower Km for sedoheptulose-7-phosphate (S7P) than for ribose-5-phosphate (R5P); or any combination thereof. Introduction of exogenous transketolase or the variant with specific substrates selectivity, controls the reaction directionality from G3P + S7P to X5P + R5P independent of pentose phosphate pathway.

In some embodiments, the exogenous transketolase and the variant possesses a ratio of Km for G3P or S7P to Km for X5P or R5P that is less than 1:1. In some embodiments, the ratio is less than 1:1.5, 1:2, 1:2.5, 1:3, 1:3.5, 1:4, 1:4.5 or 1:5.

In some embodiments, the exogenous transketolase and the variant possesses one or more of: (a) a ratio of Km for G3P to Km for X5P that is less than 1:1, e.g., less than 1:1.5, 1:2, 1:2.5, 1:3, 1:3.5, 1:4, 1:4.5 or 1:5; (b) a ratio of Km for G3P to Km for R5P that is less than 1:1, e.g., less than 1:1.5, 1:2, 1:2.5, 1:3, 1:3.5, 1:4, 1:4.5 or 1:5; (c) a ratio of Km for S7P to Km for X5P that is less than 1:1, e.g., less than 1:1.5, 1:2, 1:2.5, 1:3, 1:3.5, 1:4, 1:4.5 or 1:5; (d) a ratio of Km for S7P to Km for R5P that is less than 1:1, e.g., less than 1:1.5, 1:2, 1:2.5, 1:3, 1:3.5, 1:4, 1:4.5 or 1:5; or any combination thereof.

In some embodiments, the exogenous transketolase or its variant possesses a ratio of Km for S7P to Km for X5P that is less than 1:1, e.g., less than 1:1.5, 1:2, 1:2.5, 1:3, 1:3.5, 1:4, 1:4.5 or 1:5, e.g., less than 1:4.

In some embodiments, the exogenous transketolase and the variant at least possesses a lower Km for fructose-6-phosphate (F6P) than for xylulose 5-phosphate (X5P), and optionally further possesses a lower Km for sedoheptulose-7-phosphate (S7P) than for xylulose 5-phosphate (X5P).

In some embodiments, the exogenous transketolase or its variant possesses a ratio of Km for F6P to Km for X5P that is less than 1:1 (e.g., 1:4) and a ratio of Km for S7P to Km for X5P that is less than 1:1 (e.g., 1:4).

In some embodiments, the exogenous transketolase or its variant possesses a Km for F6P of about 0.02~0.04 mM (e.g., about 0.025~0.035 mM, such as about 0.029 mM), a Km for S7P of about 0.02~0.04 mM (e.g., about 0.025~0.035 mM, such as about 0.031 mM), and a Km for X5P of about 0.1~0.6 mM (e.g., about 0.1~0.55 mM, such as 0.145 mM).

### II-B. kcat value

In some embodiments, the exogenous transketolase or its variant possesses one or more of: (i) a higher kcat for glyceraldehyde-3-phosphate (G3P) than for erythrose-4-phosphate (E4P); (ii) a higher kcat for glyceraldehyde-3-phosphate (G3P) than for xylulose 5-phosphate (X5P); (iii) a higher kcat for fructose-6-phosphate (F6P) than for erythrose-4-phosphate (E4P); (iv) a higher kcat for fructose-6-phosphate (F6P) than for xylulose 5-phosphate (X5P); or any combination thereof.

In some embodiments, the exogenous transketolase and the variant at least possesses a higher kcat for fructose-6-phosphate (F6P) than for xylulose 5-phosphate (X5P).

In some embodiments, the exogenous transketolase and the variant possesses a ratio of kcat for G3P or F6P to kcat for E4P or X5P that is higher than 1:1. In some embodiments, the ratio is higher than 1.5:1, 2:1, 2.5:1, 3:1, 3.5:1, 4:1, 4.5:1 or 5:1.

In some embodiments, the exogenous transketolase and the variant possesses one or more of: (i) a ratio of kcat for G3P to kcat for E4P that is higher than 1:1, e.g., higher than 1.5:1, 2:1, 2.5:1, 3:1, 3.5:1, 4:1, 4.5:1 or 5:1; (ii) a ratio of kcat for G3P to kcat for X5P that is higher than 1:1, e.g., higher than 1.5:1, 2:1, 2.5:1, 3:1, 3.5:1, 4:1, 4.5:1 or 5:1; (iii) a ratio of kcat for F6P to kcat for E4P that is higher than 1:1, e.g., higher than 1.5:1, 2:1, 2.5:1, 3:1, 3.5:1, 4:1, 4.5:1 or 5:1; (iv) a ratio of kcat for F6P to kcat for X5P that is higher than 1:1, e.g., higher than 1.5:1, 2:1, 2.5:1, 3:1, 3.5:1, 4:1, 4.5:1 or 5:1; or any combination thereof.

In some embodiments, the exogenous transketolase and the variant at least possesses a ratio of kcat for F6P to kcat for X5P that is higher than 1:1, e.g., higher than 1.5:1, 2:1, 2.5:1, 3:1, 3.5:1, 4:1, 4.5:1 or 5:1, e.g., higher than 4:1.

In some embodiments, the exogenous transketolase and the variant as described herein further possesses a higher kcat for glyceraldehyde-3-phosphate (G3P) or sedoheptulose-7-phosphate (S7P) than for xylulose 5-phosphate (X5P) or ribose-5-phosphate (R5P). In some embodiments, the exogenous transketolase and the variant possesses one or more of: (a) a higher kcat for glyceraldehyde-3-phosphate (G3P) than for xylulose 5-phosphate (X5P); (b) a higher kcat for glyceraldehyde-3-phosphate (G3P) than for ribose-5-phosphate (R5P); (c) a higher kcat for sedoheptulose-7-phosphate (S7P) than for xylulose 5-phosphate (X5P); (d) a higher kcat for sedoheptulose-7-phosphate (S7P) than for ribose-5-phosphate (R5P); or any combination thereof. Introduction of exogenous transketolase or the variant with specific substrates selectivity, controls the reaction directionality from G3P + S7P to X5P + R5P independent of pentose phosphate pathway.

In some embodiments, the exogenous transketolase and the variant possesses a ratio of kcat for G3P or S7P to kcat for X5P or R5P that is higher than 1:1. In some embodiments, the ratio is higher than 1.5:1, 2:1, 2.5:1, 3:1, 3.5:1, 4:1, 4.5:1 or 5:1.

In some embodiments, the exogenous transketolase and the variant possesses one or more of: (a) a ratio of kcat for G3P to kcat for X5P that is higher than 1:1, e.g., higher than 1.5:1, 2:1, 2.5:1, 3:1, 3.5:1, 4:1, 4.5:1 or 5:1; (b) a ratio of kcat for G3P to kcat for R5P that is higher than 1:1, e.g., higher than 1.5:1, 2:1, 2.5:1, 3:1, 3.5:1, 4:1, 4.5:1 or 5:1; (c) a ratio of kcat for S7P to kcat for X5P that is higher than 1:1, e.g., higher than 1.5:1, 2:1, 2.5:1, 3:1, 3.5:1, 4:1, 4.5:1 or 5:1; (d) a ratio of kcat for S7P to kcat for R5P that is higher than 1:1, e.g., higher than 1.5:1, 2:1, 2.5:1, 3:1, 3.5:1, 4:1, 4.5:1 or 5:1; or any combination thereof.

In some embodiments, the exogenous transketolase or its variant possesses a ratio of kcat for S7P to kcat for X5P that is higher than 1:1, e.g., higher than 1.5:1, 2:1, 2.5:1, 3:1, 3.5:1, 4:1, 4.5:1 or 5:1, e.g., higher than 4:1.

In some embodiments, the exogenous transketolase and the variant at least possesses a higher kcat for fructose-6-phosphate (F6P) than for xylulose 5-phosphate (X5P), and optionally further possesses a higher kcat for sedoheptulose-7-phosphate (S7P) than for xylulose 5-phosphate (X5P).

In some embodiments, the exogenous transketolase or its variant possesses a ratio of kcat for F6P to kcat for X5P that is higher than 1:1 (e.g., 4:1) and a ratio of kcat for S7P to kcat for X5P that is higher than 1:1 (e.g., 4:1).

### III. Exemplary transketolases

Exemplary sequences and sources for exogenous transketolases are provided below.

In some embodiments, the exogenous transketolase or its variant is derived from *Leishmania mexicana*, *Scheffersomyces stipites*, *Debaryomyces hansenii*, *Suhomyces tanzawaensis*, *Candida subhashii*, *Candida tenuis*, *Hyphopichia burtonii*, *Spathaspora passalidarum*, *Candida intermedia*, or *Pichia sorbitophila*.

In some embodiments, the exogenous transketolase is selected from: the *Scheffersomyces stipites* transketolase of SEQ ID NO: 1 or a variant thereof. In some embodiments, the *Scheffersomyces stipites* transketolase of SEQ ID NO: 1 possesses a Km for F6P of about 0.029 mM, a Km for S7P of about 0.031 mM, and a Km for X5P of about 0.145 mM.

In some embodiments, the exogenous transketolase is selected from: the *Scheffersomyces stipites* transketolase variant of SEQ ID NO: 12. In some embodiments, the *Scheffersomyces stipites* transketolase variant of SEQ ID NO: 12 possesses a Km for F6P of about 0.029 mM, a Km for S7P of about 0.031 mM, and a Km for X5P of about 0.517 mM.

In some embodiments, the exogenous transketolase is selected from: the *Leishmania mexicana* transketolase of SEQ ID NO: 2 or a variant thereof.

In some embodiments, the exogenous transketolase is selected from: the *Debaryomyces hansenii* transketolase of SEQ ID NO: 3 or a variant thereof.

In some embodiments, the exogenous transketolase is selected from: the *Suhomyces tanzawaensis* transketolase of SEQ ID NO: 4 or a variant thereof.

In some embodiments, the exogenous transketolase is selected from: the *Candida subhashii* transketolase of SEQ ID NO: 5 or a variant thereof.

In some embodiments, the exogenous transketolase is selected from: the *Candida tenuis* transketolase of SEQ ID NO: 6 or a variant thereof.

In some embodiments, the exogenous transketolase is selected from: the *Hyphopichia burtonii* transketolase of SEQ ID NO: 7 or a variant thereof.

In some embodiments, the exogenous transketolase is selected from: the *Spathaspora passalidarum* transketolase of SEQ ID NO: 8 or a variant thereof.

In some embodiments, the exogenous transketolase is selected from: the *Candida intermedia* transketolase of SEQ ID NO: 9 or a variant thereof.

In some embodiments, the exogenous transketolase is selected from: the *Pichia sorbitophila* transketolase of SEQ ID NO: 10 or a variant thereof.

In some embodiments, the exogenous transketolase is selected from: the *Pichia sorbitophila* transketolase of SEQ ID NO: 11 or a variant thereof.

In some embodiments, the exogenous transketolase comprises the amino acid sequence of any one of SEQ ID NOs: 1-12.

Although exemplary sequences and sources for exogenous transketolases are provided herein, the invention is by no means limited to these sequences and sources, it also encompasses variants. The term "variant" includes proteins whose sequence varies from the sequence of a reference protein exemplified herein. For example, the variant possesses substantially the same specific substrates selectivity, catalytic activity, and/or catalytic efficiency as the reference exogenous transketolase exemplified herein. Such variant may be referred to herein as "functionally equivalent variant".

In some embodiments, the functionally equivalent variant at least possesses the Km value as defined in Part II-A above.

In some embodiments, the functionally equivalent variant may share high structural similarity with the reference exogenous transketolase exemplified herein.

In some embodiments, the functionally equivalent variant may have at least 90% (e.g., at least 91%, at least 92%, at least 92%, at least 93%, at least 94%, at least 95%, at least 96%, at least 97%, at least 98%, or at least 99%) sequence identity compared to the reference transketolase exemplified herein.

In some embodiments, the functionally equivalent variant may have one or more substitutions (e.g., one substitution) compared to the reference transketolase exemplified herein.

### Pentose phosphate pathway

The engineered microbial cell as described herein comprises blocked or reduced flux through pentose phosphate pathway. The pentose phosphate pathway (HMP) is a process that breaks down glucose-6-phosphate into NADPH and pentoses (5-carbon sugars) for use in downstream biological processes. There are two distinct phases in the pathway: the oxidative phase and the non-oxidative phase. The first is the oxidative phase in which glucose-6-phosphate is converted to ribulose-5-phosphate. The second phase of this pathway is the non-oxidative synthesis of 5-carbon sugars.

The phrase "blocked or reduced flux" is intended to mean to decrease the extent or flow of metabolic carbon through or to a desired pathway, pathway product, intermediate, or bioderived compound. The decrease can be relative to a predetermined baseline (e.g. the level of a wild-type cell or a parental cell) of a pathway product, intermediate or bioderived compound. For example, a blocked or reduced flux through pentose phosphate pathway can be achieved with attenuated pentose phosphate pathway enzyme(s), as compared to with functional ones. As used herein, the term "pathway enzyme" refers to an enzyme that functions in the desired pathway leading to the production of a naturally occurring molecule.

In some embodiments, the engineered microbial cell comprises blocked or reduced oxidative phase of pentose phosphate pathway, which can be achieved, for example, with reduced activity of enzyme(s) involved in the oxidative phase.

In some embodiments, the engineered microbial cell comprises reduced activity of 6-phosphogluconate dehydrogenase and/or glucose-6-phosphate dehydrogenase, e.g., achieved by deleting, knocking out or knocking down the endogenous gene(s) encoding the enzyme(s), or by modifying or replacing the endogenous gene(s) encoding the enzyme(s) to contain a rare codon.

In some embodiments, the endogenous gene encoding 6-phosphogluconate dehydrogenase is deleted, knocked out or knocked down or modified or replaced to contain a rare codon, which includes *gnd* gene or its species homologs (e.g., orthologs, or paralogs). In some embodiments where the microbial cell is *E. coli*, the endogenous *gnd* gene is deleted, knocked out or knocked down.

In some embodiments, the endogenous gene encoding glucose-6-phosphate dehydrogenase is deleted, knocked out or knocked down, which includes *zwf* gene or its species homologs (e.g., orthologs, or paralogs). In some embodiments where the microbial cell is *E. coli*, the endogenous *zwf* gene is deleted, knocked out or knocked down.

### Other genetic modifications (1)

In any of the aforementioned engineered microbial cells, one or more further genetic modifications that facilitate or optimize the biosynthesis of aromatic amino acids can also be introduced. For example, such genetic modifications include but are not limited to: (i) enhanced flux through the nitrogen assimilation pathway, (ii) reduced activity of endogenous transketolase, (iii) reduced activity of endogenous transaldolase, and/or (iv) blocked or attenuated flux of the TCA cycle.

### I. Nitrogen assimilation pathway

In some embodiments, the engineered microbial cell as described herein may further comprise enhanced flux through nitrogen assimilation pathway. The phrase "enhanced flux" is intended to mean to intensify, increase, or further improve the extent or flow of metabolic carbon through or to a desired pathway, pathway product, intermediate, or bioderived compound. The intensity, increase or improvement can be relative to a predetermined baseline (e.g. the level of wild-type cell or parent cell) of a pathway product, intermediate or bioderived compound. For example, an enhanced flux through nitrogen assimilation pathway can be achieved by overexpression of glutamate dehydrogenase and/or glutamine synthetase, as compared to in a parent or wild-type microbial cell.

In some embodiments, the engineered microbial cell comprises one or more of: (i) increased activity of glutamate dehydrogenase and/or glutamine synthetase, (ii) reduced activity of glutamate synthase, or any combination thereof.

In some embodiments, the reduced activity of glutamate synthase can be achieved, for example, by deleting, knocking out or knocking down the endogenous gene encoding glutamate synthase, or by modifying or replacing the endogenous gene encoding glutamate synthase to contain a rare codon, which includes *gltBD* gene or its species homologs (e.g., orthologs, or paralogs). In some embodiments where the microbial cell is *E. coli*, the endogenous *gltBD* gene is deleted, knocked out or knocked down.

In some embodiments, the increased activity of glutamate dehydrogenase and/or glutamine synthetase can be achieved, for example, by overexpression of the endogenous gene(s) encoding the enzyme(s), which include *gdhA* and *glnA* genes or their species homologs (e.g., orthologs, or paralogs). In some embodiments where the microbial cell is *E. coli*, the endogenous *gdhA* and *glnA* genes are overexpressed. In certain embodiments, overexpression may occur at the transcriptional level, translational level, or both, which may be due to altered regulatory control (e.g., use of a strong promoter) or an increase in copy number or both.

In some embodiments, when the microbial cell is *Escherichia coli*, the endogenous *gltBD* gene is deleted, knocked out, or knocked down, and the endogenous *gdhA* and *glnA* genes are overexpressed.

### II. Engineering endogenous transketolase

In some embodiments, the engineered microbial cell as described above further comprises reduced activity of endogenous transketolase. The reduced activity can be achieved by any methods known in the art, including without limitation, introduction of gene disruption, e.g., complete gene deletion or knockout by gene editing systems, or knockdown by using an oligonucleotide that binds to the gene coding for it, or to its mRNA, or modification or replacement of an endogenous gene to contain a rare codon, leading to a temporary change in expression.

In some embodiments, the endogenous gene encoding transketolase is deleted, knocked out or knocked down or is modified or replaced to contain a rare codon. The endogenous gene encoding transketolase includes *tktA* gene and/or *tktB* gene or its species homologs (e.g., orthologs, or paralogs). In some embodiments where the microbial cell is *E. coli*, the endogenous *tktA* gene is deleted, knocked out or knocked down, or the endogenous *tktA* gene is modified or replaced to contain a rare codon. In some embodiments where the microbial cell is *E. coli*, the endogenous *tktB* gene is deleted, knocked out or knocked down, or the endogenous *tktB* gene is modified or replaced to contain a rare codon. In some embodiments where the microbial cell is *E. coli*, the endogenous *tktA* and *tktB* genes are deleted, knocked out or knocked down, or the endogenous *tktA* and *tktB* genes are modified or replaced to contain a rare codon.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally further comprises: (iii) deletion, knockout, or knockdown of endogenous *tktA* gene and/or *tktB* gene.

### III. Engineering endogenous transaldolase

In some embodiments, the engineered microbial cell as described above further comprises reduced activity of endogenous transaldolase. The reduced activity can be achieved by any methods known in the art, including without limitation, introduction of gene disruption, e.g., complete gene deletion or knockout by gene editing systems, or knockdown by using an oligonucleotide that binds to the gene coding for it, or to its mRNA, leading to a temporary change in expression.

In some embodiments, the endogenous gene encoding transaldolase is deleted, knocked out or knocked down, or the endogenous gene encoding transaldolase is modified or replaced to contain a rare codon. The endogenous gene encoding transaldolase includes *talA* gene and/or *talB* gene or its species homologs (e.g., orthologs, or paralogs). In some embodiments where the microbial cell is *E. coli*, the endogenous *talA* gene is deleted, knocked out or knocked down. In some embodiments where the microbial cell is *E. coli*, the endogenous *talB* gene is deleted, knocked out or knocked down. In some embodiments where the microbial cell is *E. coli*, the endogenous *talA* and *talB* genes are deleted, knocked out or knocked down.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally further comprises: (iii) deletion, knockout, or knockdown of endogenous *talA* gene and/or *talB* gene.

### IV. TCA cycle

In some embodiments, the engineered microbial cell as described herein comprises blocked or attenuated flux of the TCA cycle. The tricarboxylic acid cycle (TCA) is a series of biochemical reactions that are used to generate energy in the form of ATP through the oxidation of acetyl-CoA derived from carbohydrates, fats, and proteins.

The phrase "blocked or attenuated flux" is intended to mean to decrease the extent or flow of metabolic carbon through or to a desired pathway, pathway product, intermediate, or bioderived compound. The decrease can be relative to a predetermined baseline (e.g. the level of a wild-type cell or a parental cell) of a pathway product, intermediate or bioderived compound. For example, a blocked or attenuated flux through TCA pathway can be achieved by attenuated TCA pathway enzyme(s), as compared to with functional ones. As used herein, the term "TCA pathway enzyme" refers to an enzyme that functions in the TCA pathway.

In some embodiments, the engineered microbial cell comprises reduced flux of acetyl-CoA into TCA cycle, e.g., achieved by reduced activity of pyruvate dehydrogenase and/or citrate synthase.

In some embodiments, the engineered microbial cell comprises reduced activity of pyruvate dehydrogenase and/or citrate synthase, e.g. achieved by deleting, knocking out or knocking down the endogenous gene encoding pyruvate dehydrogenase and/or citrate synthase, or by modifying or replacing the endogenous gene encoding pyruvate dehydrogenase and/or citrate synthase to contain a rare codon.

In some embodiments, the endogenous gene encoding pyruvate dehydrogenase is deleted, knocked out or knocked down, or the endogenous gene encoding pyruvate dehydrogenase is modified or replaced to contain a rare codon. Such endogenous genes include, for example, *lpd*, *aceF*, *aceE* genes or their species homologs (e.g., orthologs or paralogs). In some embodiments where the microbial cell is *Escherichia coli*, one or more genes selected from *lpd*, *aceF*, and *aceE* are deleted, knocked out, or knocked down.

In some embodiments, the endogenous gene encoding citrate synthase is deleted, knocked out or knocked down, or the endogenous gene encoding citrate synthase is modified or replaced to contain a rare codon. Such endogenous genes include, for example *gltA* gene or its species homologs (e.g., orthologs, or paralogs). In some embodiments where the microbial cell is *E. coli*, the endogenous *gltA* gene is modified or replaced to contain a rare codon.

Different species or their cells may exhibit variations in codon preference for encoding specific amino acids. Codons that are most frequently utilized are referred to as optimal codons, while those that are rarely utilized are termed a rare codon or low-usage codons. Therefore, as used herein, the term "rare codon(s)" refers to a codon encoding an amino acid that is employed with lower frequency during gene expression in a particular species or cell. For instance, when expressing a heterologous gene in *Escherichia coli*, a rare codon that is infrequently used by *E. coli* can reduce the availability of homologous tRNAs, thereby diminishing the efficiency and level of protein expression. For a given species or cell, a person skilled in the art can readily identify a rare codon using various analytical tools, such as bioinformatics tools.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally the engineered microbial cell described above further comprises: (iii) modification or replacement of endogenous *gltA* gene to contain a rare codon.

### Other genetic modifications (2)

In any of the aforementioned engineered microbial cells, one or more further genetic modifications that facilitate or optimize the biosynthesis of aromatic amino acids can also be introduced. For example, strategies that allow higher PEP availability for the aromatic amino acid biosynthesis can be employed, including, for instance, (i) limiting carbon flow from phosphoenolpyruvate (PEP) to oxaloacetate, (ii) the replacement of glucose transport and phosphorylation capabilities of phosphotransferase (PTS) transport system by alternative enzymes, and/or (iii) improving the recycling of pyruvate (PYR) to PEP. For example, the approach to reduce the accumulation of acetic acid and/or lactic acid can be employed, to overcome the inhibition on microbial growth.

### I. Oxaloacetate biosynthesis pathway

In some embodiments, the engineered microbial cell as described above further comprises blocked or reduced oxaloacetate biosynthesis pathway, to limit carbon flow from phosphoenolpyruvate (PEP) to oxaloacetate.

In some embodiments, the engineered microbial cell comprises reduced activity of phosphoenolpyruvate carboxylase. In some embodiments, the reduced activity of phosphoenolpyruvate carboxylase can be achieved, for example, by deleting, knocking out or knocking down the endogenous gene encoding phosphoenolpyruvate carboxylase, or by modifying or replacing the endogenous gene encoding phosphoenolpyruvate carboxylase to contain a rare codon. Such endogenous gene includes *ppc* gene or its species homologs (e.g., orthologs, or paralogs). In some embodiments where the microbial cell is *E. coli*, the endogenous *ppc* gene is deleted, knocked out or knocked down.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally the engineered microbial cell described above further comprises: (iii) deletion, knockout, or knockdown of endogenous *ppc* gene.

### II. PTS transport system

Phosphotransferase (PTS) transport system is widely found in bacteria, fungi and some archaea as the main system for the translocation and phosphorylation of glucose from the periplasmic space to the cytoplasmic environment, consuming one PEP molecule which is converted to pyruvate (PYR). To prevent loss of PEP towards the PTS system and improve intracellular PEP-availability, the naturally occurring PTS system can be disabled. Consequently, in order to maintain the capability of the engineered microbial cell to use a certain sugar (e.g. glucose) as carbon- and energy source, a phosphoenolpyruvate: sugar phosphotransferase independent importer needs to be expressed or overexpressed. Further, the expression or overexpression of a kinase, capable to phosphorylate said certain sugar, may also be required or decreased and/or diminished, in order to realize metabolization of said sugar or its accumulation in an unphosphorylated form within the cell, respectively.

In some embodiments, the engineered microbial cell as described above further comprises disabled naturally occurring phosphotransferase (PTS) transport system. In some embodiments, the disabled naturally occurring PTS transport system comprises reduced activity of one or more PTS system protein(s). In some embodiments, the PTS system protein(s) comprises soluble non-sugar-specific protein components Enzyme I (EI, *ptsI*) and histidine protein (HPr, *ptsH*), soluble glucose-specific enzyme IIA^{Glc} (*crr*), and membrane-integral glucose-specific permease IICB^{Glc} (*ptsG*).

In some embodiments, the disabled PTS transport system is achieved by deleting, knocking out or knocking down one or more gene(s) selected from *ptsH*, *ptsI*, *crr*, *ptsG* or their species homologs (e.g., orthologs or paralogs), or any combination thereof. In the case of *E. coli*, one or more gene(s) selected from *ptsH*, *ptsI*, *crr*, *ptsG* is deleted, knocked out or knocked down.

In some embodiments, the disabled PTS transport system is achieved by deleting, knocking out or knocking down *ptsI* and/or *ptsG* genes or their species homologs (e.g., orthologs or paralogs). In the case of *E. coli*, *ptsI* and/or *ptsG* genes is deleted, knocked out or knocked down.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally the engineered microbial cell described above further comprises: (iii) deletion, knockout, or knockdown of endogenous *ptsH, ptsI, crr, ptsG* genes, or any combination thereof.

In some embodiments, the engineered microbial cell further comprises increased activity of at least one non-PTS sugar transporter. Suitable non-PEP-PTS transporters, capable of transferring monosaccharides (glucose and/or fructose and/or galactose and/or N-acetylglucosamine and/or N-acetylneuraminic acid and/or fucose) into a microbial cell, are known to a skilled person in the art, e.g., *Glf* of *Zymomonas mobilis*.

In some embodiments, the increased activity is achieved by overexpression of endogenous non-PTS sugar transporter.

In some embodiments, the increased activity is achieved by heterologous expression of exogenous non-PTS sugar transporter, such as glucose facilitator (Glf) of *Zymomonas mobilis*.

In some embodiments, the engineered microbial cell further comprises increased activity of glucokinase. In some embodiments, the increased activity of glucokinase can be achieved, for example, by overexpression of the endogenous gene encoding the enzyme, which include *glk* gene or its species homologs (e.g., orthologs or paralogs), or heterologous expression of exogenous *glk* gene, e.g., from *Zymomonas mobilis*. In some embodiments where the microbial cell is *E. coli*, the endogenous *glk* gene is overexpressed, or the exogenous *glk* gene (e.g., from *Zymomonas mobilis*) is introduced.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally the engineered microbial cell described above further comprises: (iii) overexpression of endogenous *glf* gene or *glk* gene, or heterologous expression of exogenous *glf* gene or *glk* gene.

In some embodiments, the engineered microbial cell comprises disabled naturally occurring phosphotransferase (PTS) transport system, and increased activity of at least one non-PTS sugar transporter. In some embodiments where the microbial cell is *E. coli*, one or more gene(s) selected from *ptsH*, *ptsI*, *crr*, *ptsG* , or any combination thereof is deleted, knocked out or knocked down, or is modified or replaced to cantina a rare codon; and the endogenous *glf* gene is overexpressed or the exogenous *glk* gene (e.g., from *Zymomonas mobilis*) is heterologously expressed.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally the engineered microbial cell described above further comprises: (iii) overexpression of endogenous *glf* gene or *glk* gene, or heterologous expression of exogenous *glf* gene or *glk* gene, along with deletion, knockout, or knockdown of endogenous *ptsH*, *ptsI*, *crr*, *ptsG*, or any combination thereof.

### III. Acetic acid and/or lactic acid production pathways

The accumulation of acetic acid and/or lactic acid results in inhibitory effect on microbial growth and consequent limitation on the production efficiency of aromatic amino acids. Thus, reducing the accumulation of acetic acid and/or lactic acid would facilitate the production of aromatic amino acids.

In some embodiments, the engineered microbial cell as described above further comprises blocked or reduced pathways for production of acetic acid and/or lactic acid.

In some embodiments, the engineered microbial cell comprises reduced activity of one or more enzymes selected from phosphate acetyltransferase, pyruvate dehydrogenase, lactate dehydrogenase, or any combination thereof. In some embodiments, the reduced activity can be achieved, for example, by deleting, knocking out or knocking down one or more endogenous gene(s) encoding the one or more enzyme(s), which includes *pta*, *poxB*, *ldhA* genes or their species homologs (e.g., orthologs or paralogs). In some embodiments where the microbial cell is *E. coli*, one or more gene(s) selected from *pta*, *poxB* and/or *ldhA* is deleted, knocked out or knocked down.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally the engineered microbial cell described above further comprises: (iii) deletion, knockout, or knockdown of endogenous *pta*, *poxB*, *ldhA*, or any combination thereof.

### IV. Recycling to PEP

The flow of carbon to phosphoenolpyruvate (PEP) can be enhanced to increase the availability of PEP for the aromatic amino acid biosynthesis.

In some embodiments, the engineered microbial cell as described above further comprises an enhanced phosphoenolpyruvate (PEP) biosynthesis pathway.

In some embodiments, the enhanced PEP biosynthesis pathway comprises increased activity of phosphoenolpyruvate synthase to enhance conversion from pyruvate to PEP. The increased activity can be achieved, for example, by overexpression of the endogenous gene encoding the enzyme or heterologous expression of exogenous gene encoding the enzyme, which includes *ppsA* gene or its species homologs (e.g., orthologs or paralogs). In some embodiments where the microbial cell is *E. coli*, the endogenous *ppsA* gene is overexpressed. In some embodiments where the microbial cell is *E. coli*, the exogenous *ppsA* gene is heterologous expressed.

In some embodiments, the enhanced PEP biosynthesis pathway comprises increased activity of pyruvate carboxylase and PEP carboxykinase to enhance conversion from pyruvic acid to PEP. The increased activity can be achieved, for example, by the combined overexpression of both endogenous or exogenous *pyc* genes and endogenous or exogenous *pck* genes, for example, achieved by overexpression of the endogenous gene encoding the enzymes, which include *pyc* and *pck* gene or their species homologs (e.g., orthologs or paralogs), or heterologous expression of exogenous *pyc* and *pck* genes, e.g., exogenous *pyc* gene from *Cytobacillus firmus*. For example, in some embodiments, the increased activity can be achieved by overexpression of endogenous *pyc* gene encoding pyruvate carboxylase or its species homologs (e.g., orthologs or paralogs), or by heterologous expression of exogenous *pyc* gene, e.g., exogenous *pyc* gene from *Cytobacillus firmus*. In some embodiments, the increased activity can be achieved by overexpression of endogenous *pck* gene encoding PEP carboxykinase or its species homologs (e.g., orthologs or paralogs), or by heterologous expression of exogenous *pck* gene. In some embodiments where the microbial cell is *E. coli*, the endogenous *pyc* and *pck* genes are overexpressed. In some embodiments where the microbial cell is *E. coli*, the endogenous *pck* gene is overexpressed and exogenous *pyc* gene (e.g., from *Cytobacillus firmus*) is introduced.

In some embodiments, the engineered microbial cell as described above comprises: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally the engineered microbial cell described above further comprises: (iii) overexpression of endogenous *ppsA, pyc, pck* genes, or any combination thereof, or heterologous expression of exogenous *ppsA, pyc, pck* genes, or any combination thereof.

### Exemplary engineered microbes

Exemplary embodiments of engineered microbial cells provided herein include, but are not limited to, the following.

In some embodiments, provided herein is an engineered microbial cell, wherein at least two or more optional modifications may be combined to obtain an engineered microbial cell with desired properties.

In some embodiments, provided herein is an engineered microbial cell comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and
(ii) deletion, knockout, or knockdown of endogenous *gnd* gene or *zwf* gene;
optionally the engineered microbial cell described above further comprises any one of the following:
(iii) deletion, knockout, or knockdown of endogenous *gltBD* gene;
(iv) deletion, knockout, or knockdown of endogenous *tktA* gene and/or *tktB* gene;
(v) deletion, knockout, or knockdown of endogenous *talA* gene and/or *talB* gene;
(vi) any combination of (iii) to (v).

In some embodiments, provided herein is an engineered microbial cell comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and
(ii) deletion, knockout, or knockdown of endogenous *gnd* gene and/or *zwf* gene;
optionally the engineered microbial cell described above further comprises any one of the following:
(iii) deletion, knockout, or knockdown of endogenous *gltBD* gene;
(iv) deletion, knockout, or knockdown of endogenous *tktA* gene and/or *tktB* gene;
(v) deletion, knockout, or knockdown of endogenous *talA* gene and/or *talB* gene;
(vi) modification or replacement of endogenous *gltA* gene to contain a rare codon;
(vii) deletion, knockout, or knockdown of endogenous *ppc* gene;
(viii) deletion, knockout, or knockdown of endogenous *ptsH*, *ptsI*, *crr*, *ptsG* gene, or any combination thereof;
(ix) overexpression of endogenous *glf* gene or *glk* gene or heterologous expression of exogenous *glf* gene or *glk* gene;
(x) deletion, knockout, or knockdown of endogenous *pta*, *poxB*, *ldhA* gene, or any combination thereof;
(xi) overexpression of endogenous *ppsA, pyc, pck* gene or any combination thereof; or heterologous expression of exogenous *ppsA, pyc, pck* gene or any combination thereof;
(xii) any combination of (iii) to (xi).

In some embodiments, provided herein is an engineered microbial cell comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and
(ii) deletion, knockout, or knockdown of endogenous *gnd* gene and/or *zwf* gene;
(iii) deletion, knockout, or knockdown of endogenous *talA* gene and/or *talB* gene;
optionally the engineered microbial cell described above further comprises any one of the following:
(iv) deletion, knockout, or knockdown of endogenous *gltBD* gene;
(v) deletion, knockout, or knockdown of endogenous *tktA* gene and/or *tktB* gene;
(vi) modification or replacement of endogenous *gltA* gene to contain a rare codon;
(vii) deletion, knockout, or knockdown of endogenous *ppc* gene;
(viii) deletion, knockout, or knockdown of endogenous *ptsH*, *ptsI*, *crr*, *ptsG* gene, or any combination thereof;
(ix) overexpression of endogenous *glf* gene or *glk* gene or heterologous expression of exogenous *glf* gene or *glk* gene;
(x) deletion, knockout, or knockdown of endogenous *pta*, *poxB*, *ldhA* gene, or any combination thereof;
(xi) overexpression of endogenous *ppsA, pyc, pck* gene or any combination thereof or heterologous expression of exogenous *ppsA, pyc, pck* gene or any combination thereof;
(xii) any combination of (iv) to (xi).

In some embodiments, provided herein is an engineered microbial cell comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and
(ii) deletion, knockout, or knockdown of endogenous *gnd* gene and/or *zwf* gene;
(iii) deletion, knockout, or knockdown of endogenous *gltBD* gene;
optionally the engineered microbial cell described above further comprises any one of the following:
(iv) deletion, knockout, or knockdown of endogenous *tktA* gene and/or *tktB* gene;
(v) deletion, knockout, or knockdown of endogenous *talA* gene and/or *talB* gene;
(vi) modification or replacement of endogenous *gltA* gene to contain a rare codon;
(vii) deletion, knockout, or knockdown of endogenous *ppc* gene;
(viii) deletion, knockout, or knockdown of endogenous *ptsH*, *ptsI*, *crr*, *ptsG* gene, or any combination thereof;
(ix) overexpression of endogenous *glf* gene or *glk* gene or heterologous expression of exogenous *glf* gene or *glk* gene;
(x) deletion, knockout, or knockdown of endogenous *pta*, *poxB*, *ldhA* gene, or any combination thereof;
(xi) overexpression of endogenous *ppsA, pyc, pck* gene or any combination thereof; or heterologous expression of exogenous *ppsA, pyc, pck* gene or any combination thereof;
(xii) any combination of (iv) to (xi).

In some embodiments, provided herein is an engineered microbial cell comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and
(ii) deletion, knockout, or knockdown of endogenous *gnd* gene and/or *zwf* gene;
(iii) deletion, knockout, or knockdown of endogenous *gltBD* gene;
(iv) deletion, knockout, or knockdown of endogenous *tktA* gene and/or *tktB* gene;
(v) deletion, knockout, or knockdown of endogenous *talA* gene and/or *talB* gene;
optionally the engineered microbial cell described above further comprises any one of the following:
(vi) modification or replacement of endogenous *gltA* gene to contain a rare codon;
(vii) deletion, knockout, or knockdown of endogenous *ppc* gene;
(viii) deletion, knockout, or knockdown of endogenous *ptsH*, *ptsI*, *crr*, *ptsG* gene, or any combination thereof;
(ix) overexpression of endogenous *glf* gene or *glk* gene or heterologous expression of exogenous *glf* gene or *glk* gene;
(x) deletion, knockout, or knockdown of endogenous *pta*, *poxB*, *ldhA* gene, or any combination thereof;
(xi) overexpression of endogenous *ppsA, pyc, pck* gene or any combination thereof; or heterologous expression of exogenous *ppsA, pyc, pck* gene or any combination thereof;
(xii) any combination of (vi) to (xi).

In some embodiments, provided herein is an engineered microbial cell comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4);
(ii) deletion, knockout, or knockdown of endogenous *gnd* gene;
(iii) deletion, knockout, or knockdown of endogenous *gltBD* gene;
(iv) deletion, knockout, or knockdown of endogenous *tktA* gene and *tktB* gene;
(v) deletion, knockout, or knockdown of endogenous *talA* gene and *talB* gene;
(vi) overexpression of endogenous *ppsA* gene or heterologous expression of exogenous *ppsA*;
optionally the engineered microbial cell described above further comprises any one of the following:
(vii) modification or replacement of endogenous *gltA* gene to contain a rare codon;
(viii) deletion, knockout, or knockdown of endogenous *ppc* gene;
(ix) deletion, knockout, or knockdown of endogenous *ptsH*, *ptsI*, *crr*, *ptsG* gene, or any combination thereof;
(x) overexpression of endogenous *glf* gene or *glk* gene or heterologous expression of exogenous *glf* gene or *glk* gene;
(xi) deletion, knockout, or knockdown of endogenous *pta*, *poxB*, *ldhA* gene, or any combination thereof;
(xii) overexpression of endogenous *pyc or pck* gene or heterologous expression of exogenous *pyc or pck* gene;
(xiii) any combination of (vii) to (xii).

In some embodiments, provided herein is an engineered microbial cell comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4);
(ii) deletion, knockout, or knockdown of endogenous *gnd* gene;
(iii) deletion, knockout, or knockdown of endogenous *gltBD* gene;
(iv) deletion, knockout, or knockdown of endogenous *tktA* gene and *tktB* gene;
(v) deletion, knockout, or knockdown of endogenous *talA* gene and *talB* gene;
(vi) modification or replacement of endogenous *gltA* gene to contain a rare codon;
(vii) overexpression of endogenous *ppsA* gene or heterologous expression of exogenous *ppsA*;
optionally the engineered microbial cell described above further comprises any one of the following:
(viii) deletion, knockout, or knockdown of endogenous *ppc* gene;
(ix) deletion, knockout, or knockdown of endogenous *ptsH*, *ptsI*, *crr*, *ptsG* gene, or any combination thereof;
(x) overexpression of endogenous *glf* gene or *glk* gene or heterologous expression of exogenous *glf* gene or *glk* gene;
(xi) deletion, knockout, or knockdown of endogenous *pta*, *poxB*, *ldhA* gene, or any combination thereof;
(xii) overexpression of endogenous *pyc or pck* gene or heterologous expression of exogenous *pyc or pck* gene;
(xiii) any combination of (viii) to (xii).

In some embodiments, provided herein is an engineered microbial cell comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4);
(ii) deletion, knockout, or knockdown of endogenous *gnd* gene and *zwf* gene;
(iii) deletion, knockout, or knockdown of endogenous *gltBD* gene;
(iv) deletion, knockout, or knockdown of endogenous *tktA* gene and *tktB* gene;
(v) deletion, knockout, or knockdown of endogenous *talA* gene and *talB* gene;
(vi) overexpression of endogenous *ppsA* gene or heterologous expression of exogenous *ppsA*;
optionally the engineered microbial cell described above further comprises any one of the following:
(vii) modification or replacement of endogenous *gltA* gene to contain a rare codon;
(viii) deletion, knockout, or knockdown of endogenous *ppc* gene;
(ix) deletion, knockout, or knockdown of endogenous *ptsH*, *ptsI*, *crr*, *ptsG* gene, or any combination thereof;
(x) overexpression of endogenous *glf* gene or *glk* gene or heterologous expression of exogenous *glf* gene or *glk* gene;
(xi) deletion, knockout, or knockdown of endogenous *pta*, *poxB*, *ldhA* gene, or any combination thereof;
(xii) overexpression of endogenous *pyc or pck* gene or heterologous expression of exogenous *pyc or pck* gene;
(xiii) any combination of (vii) to (xii).

In addition, specific embodiments of the engineered microbial cell provided herein include:
Embodiment 1. An engineered microbial cell comprising: (i) heterologous expression of the exogenous transketolase or its variant as described above; and (ii) deletion, knockout, or knockdown of the endogenous gene encoding 6-phosphogluconate dehydrogenase (e.g., *gnd*) or glucose-6-phosphate dehydrogenase (e.g., *zwf*).
Embodiment 2. The engineered microbial cell of embodiment 1, the exogenous transketolase or its variant comprises the amino acid sequence of any one of SEQ ID NOs: 1-12 or functionally equivalent variants thereof.
Embodiment 3. The engineered microbial cell of embodiment 1 or 2, item (ii) comprises deletion, knockout, or knockdown of endogenous *gnd* gene or *zwf* gene or its species homologs (e.g., orthologs, or paralogs). In some cases where the microbial cell is *E. coli*, the endogenous *gnd* gene or *zwf* gene is deleted or knocked out.
Embodiment 4. The engineered microbial cell of any one of embodiments 1-3, optionally further comprising reduced activity of endogenous transketolase, e.g., achieved by deleting, knocking out or knocking down *tktA* gene.
Embodiment 5. An engineered microbial cell comprising (i) heterologous expression of the exogenous transketolase or its variant as described above; and (ii) overexpression of endogenous phosphoenolpyruvate synthase (e.g., *ppsA*) or heterologous expression of the exogenous phosphoenolpyruvate synthase (e.g., *ppsA*).
Embodiment 6. The engineered microbial cell of embodiment 5, the exogenous transketolase or its variant comprises the amino acid sequence of any one of SEQ ID NOs: 1-12 or functionally equivalent variants thereof.
Embodiment 7. The engineered microbial cell of embodiment 5 or 6, the engineered microbial cell comprises overexpression of the endogenous gene encoding phosphoenolpyruvate synthase or heterologous expression of exogenous gene encoding phosphoenolpyruvate synthase, which includes *ppsA* gene or its species homologs (e.g., orthologs, or paralogs). In some cases where the microbial cell is *E. coli*, the endogenous *ppsA* gene is overexpressed. In some cases where the microbial cell is *E. coli*, the exogenous *ppsA* gene is expressed heterologous.
Embodiment 8. An engineered microbial cell comprising (i) deletion, knockout, or knockdown of endogenous *gltBD* gene or its species homologs (e.g., orthologs, or paralogs) and (ii) overexpression of *gdhA* and *glnA* genes or their species homologs (e.g., orthologs, or paralogs). In some cases where the microbial cell is *E. coli*, the endogenous *gltBD* gene is deleted, knocked out or knocked down, and the endogenous *gdhA* and *glnA* genes are overexpressed.
Embodiment 9. An engineered microbial cell comprising: (i) heterologous expression of the exogenous transketolase or its variant as described above; (ii) deletion, knockout, or knockdown of the endogenous gene encoding 6-phosphogluconate dehydrogenase (e.g., *gnd*) or glucose-6-phosphate dehydrogenase (e.g., *zwf*); and (iii) deletion, knockout, or knockdown of endogenous gene encoding glutamate synthase (e.g., *gltBD*).
Embodiment 10. The engineered microbial cell of embodiment 9, the exogenous transketolase or its variant comprises the amino acid sequence of SEQ ID NOs: 1-12 or a sequence having at least 80% (e.g., at least 85%, at least 90%, at least 91%, at least 92%, at least 92%, at least 93%, at least 94%, at least 95%, at least 96%, at least 97%, at least 98%, or at least 99%) sequence identity thereto.
Embodiment 11. The engineered microbial cell of embodiment 9 or 10, item (ii) comprises deletion, knockout, or knockdown of endogenous *gnd* gene or *zwf* gene or its species homologs (e.g., orthologs, or paralogs). In some cases where the microbial cell is *E. coli*, the endogenous *gnd* gene or *zwf* gene (e.g., *gnd* gene) is deleted or knocked out.
Embodiment 12. The engineered microbial cell of anyone of embodiments 9-11, item (iii) comprises deletion, knockout, or knockdown of endogenous *gltBD* gene or its species homologs (e.g., orthologs, or paralogs). In some cases where the microbial cell is *E. coli*, the endogenous *gltBD* gene is deleted or knocked out.
Embodiment 13. An engineered microbial cell comprising: (i) heterologous expression of the exogenous transketolase or its variant as described above; (ii) deletion, knockout, or knockdown of the endogenous gene encoding 6-phosphogluconate dehydrogenase (e.g., *gnd*); (iii) deletion, knockout, or knockdown of endogenous gene encoding glutamate synthase (e.g., *gltBD*); (iv) overexpression of the endogenous gene encoding phosphoenolpyruvate synthase (e.g., *ppsA*) or heterologous expression of exogenous gene encoding phosphoenolpyruvate synthase (e.g., *ppsA*); (v) deletion, knockout, or knockdown of endogenous gene encoding transketolase (e.g., *tktA* and *tktB* ); (vi) deletion, knockout, or knockdown of endogenous gene encoding transaldolase (e.g., *talA* and *talB* genes).
Embodiment 14. The engineered microbial cell of embodiment 13, the exogenous transketolase or its variant comprises the amino acid sequence of any one of SEQ ID NOs: 1-12 or a sequence having at least 80% (e.g., at least 85%, at least 90%, at least 91%, at least 92%, at least 92%, at least 93%, at least 94%, at least 95%, at least 96%, at least 97%, at least 98%, or at least 99%) sequence identity thereto..
Embodiment 15. The engineered microbial cell of embodiment 13 or 14, item (ii) comprises deletion, knockout, or knockdown of endogenous *gnd* gene or its species homologs (e.g., orthologs, or paralogs). In some cases where the microbial cell is *E. coli*, the endogenous *gnd* gene is deleted or knocked out.
Embodiment 16. The engineered microbial cell of anyone of embodiments 13-15, item (iii) comprises deletion, knockout, or knockdown of endogenous *gltBD* gene or its species homologs (e.g., orthologs, or paralogs). In some cases where the microbial cell is *E. coli*, the endogenous *gltBD* gene is deleted or knocked out.
Embodiment 17. The engineered microbial cell of embodiment 13, the engineered microbial cell further comprises the modification or replacement of endogenous gene encoding citrate synthase (e.g., *gltA*) to contain a rare codon.
Embodiment 18. The engineered microbial cell of embodiment 17, the engineered microbial cell comprises the modification or replacement of endogenous *gltA* gene or its species homologs (e.g., orthologs, or paralogs). to contain a rare codon. In some cases where the microbial cell is *E. coli*, the endogenous *gltA* gene is modified or replaced to contain a rare codon.
Embodiment 19. The engineered microbial cell of embodiment 13, the engineered microbial cell further comprises the deletion, knockout, or knockdown of endogenous gene encoding glucose-6-phosphate dehydrogenase (e.g., *zwf*).
Embodiment 20. The engineered microbial cell of embodiment 19, the engineered microbial cell further comprises the deletion, knockout, or knockdown of endogenous *zwf* gene or its species homologs (e.g., orthologs or paralogs). In some cases where the microbial cell is *E. coli*, the endogenous *zwf* gene is deleted or knocked out.

### Terminal pathways

The three AAAs, tryptophan (TRP), phenylalanine (PHE) and tyrosine (TYR), are synthesized via the shikimate pathway followed by the branched aromatic amino acids biosynthesis pathway, with chorismate (CHA) serving as the last common precursor in the terminal branches.

In some embodiments, any of the aforementioned engineered microbial cells further comprises expression (e.g., heterologous expression) of genes required for TRP branch, PHE branch or TYR branch to provide the aromatic amino acid biosynthesis pathway.

The TRP branch requires the following genes that encode the enzymes needed for synthesis of TRP from CHA: *trpA*, *trpB*, *trpC*, *trpD*, and *trpE* or its feedback-resistant variants (e.g., trpE(fbr)), or their species homologs (e.g., orthologs, or paralogs)*.* In some embodiments where the target product is TRP, the engineered microbial cell disclosed herein further comprises overexpression of *trpA*, *trpB*, *trpC*, *trpD*, and *trpE* or its feedback-resistant variants (e.g., trpE(fbr)), or their species homologs (e.g., orthologs, or paralogs). In some embodiments where the microbial cell is *E. coli*, the engineered microbial cell comprises overexpression of *trpA*, *trpB*, *trpC*, *trpD*, and *trpE* or its feedback-resistant variants (e.g., trpE(fbr)). In some embodiments, overexpression of *serA*, and *aroG* or its feedback-resistant variants (e.g., aroG(fbr)) are further comprised.

The PHE branch requires the following genes that encode the enzymes needed for synthesis of TRP from CHA: pheA, tyrB or their species homologs (e.g., orthologs, or paralogs)*.* In some embodiments where the target product is PHE, the engineered microbial cell disclosed herein further comprises overexpression of pheA, tyrB, or their species homologs (e.g., orthologs, or paralogs). In some embodiments where the microbial cell is *E. coli*, the engineered microbial cell comprises overexpression of pheA, tyrB.

The TYR branch requires the following genes that encode the enzymes needed for synthesis of TYR from CHA: tyrA, tyrB or their species homologs (e.g., orthologs, or paralogs)*.* In some embodiments where the target product is TYR, the engineered microbial cell disclosed herein further comprises overexpression of tyrA, tyrB, or their species homologs (e.g., orthologs, or paralogs). In some embodiments where the microbial cell is *E. coli*, the engineered microbial cell comprises overexpression of tyrA, tyrB.

Also provided herein is a method for decreasing the production of by-products and/or increasing the yield or conversion rate of aromatic amino acid in aromatic amino acid biosynthesis, comprising introducing the genetic modification(s) comprised in any of the aforementioned engineered microbial cells to a microbial cell that naturally possesses an aromatic amino acid biosynthesis pathway.

In some embodiments, the genetic modification(s) at least comprises: (i) introduction of an exogenous transketolase possessing a ratio of Km for F6P and/or G3P to Km for E4P and/or X5P that is less than 1:1 (e.g., 1:4) and a ratio of Km for S7P and/or G3P to Km for X5P and/or R5P that is less than 1:1 (e.g., 1:4); and (ii) deletion, knockout, or knockdown of the endogenous gene encoding 6-phosphogluconate dehydrogenase or glucose-6-phosphate dehydrogenase; optionally further comprising (iii) deletion, knockout, or knockdown of endogenous gene encoding glutamate synthase. In some embodiments, the genetic modification of (i) at least comprises: introduction of an exogenous transketolase possessing a ratio of Km for F6P to Km for X5P that is less than 1:1 (e.g., 1:4) and a ratio of Km for S7P to Km for X5P that is less than 1:1 (e.g., 1:4).

### Microbial cell

The present disclosure provides genetic modifications that facilitate or optimize AAA biosynthesis. Those skilled in the art will understand that the genetic modifications as exemplified herein, are described with reference to a suitable host organism such as *E. coli* and their corresponding metabolic reactions. However, given the complete genome sequencing of a wide variety of organisms and the high level of skill in the area of genomics, those skilled in the art will readily be able to apply the teachings and guidance provided herein to essentially all other organisms. For example, the *E. coli* metabolic alterations exemplified herein can readily be applied to other species by incorporating the same or analogous encoding nucleic acid from species other than the referenced species by displacement of species homologs, e.g., orthologs or paralogs.

Orthologs and paralogs gene displacements can be determined by methods well known to those skilled in the art. For example, algorithms, such as Align, BLAST, or Clustal W, can be adopted to determine sequence identity or similarities between two compared sequences, and also determine the presence or significance of gaps in the sequence which can be assigned a weight or score. Based on such similarities, one skilled in the art can determine if the similarity is sufficiently high to indicate the proteins are related through evolution from a common ancestor.

In some embodiments, host microbial organisms can be selected from, and the engineered microbial cell can be generated in, for example, bacteria, yeast, fungi or any of a variety of other microorganisms applicable to fermentation processes, in particular aromatic amino acid biosynthesis.

In some embodiments, the host microbe naturally has an aromatic amino acid biosynthesis pathway. AAA biosynthesis is well-conserved within microorganisms, comprising the shikimate (SHK) pathway, which connects central carbon metabolism (CCM) with the biosynthesis of chorismate (CHA). AAA biosynthesis begins with the condensation of phosphoenolpyruvate (PEP) and erythrose 4-phosphate (E4P). This achieves the formation of 3-deoxy-D-arabinoheptulosonate 7-phosphate (DAHP), which is the first metabolite of the seven-step shikimate pathway for production of chorismate. Chorismate is a precursor for all three standard AAAs and a key branch point for secondary aromatic metabolites.

In some embodiments, the host microbe is a microbe that is suitable for industrial fermentation.

Exemplary bacteria include species selected from *Escherichia coli*, *Corynebacterium glutamicum*, *Bacillus subtilis*, *Bacillus licheniformis.*

*E. coli* is a particularly useful host organisms since it is a well characterized microbial organism suitable for genetic engineering. It is understood that any suitable microbial host organism can be used to introduce metabolic and/or genetic modifications to produce a desired product.

In some embodiments, the engineered microbial cell as described above is bacteria, fungi, or yeast. In some embodiments, the engineered microbial cell as described above is *Escherichia coli*.

### Methods of making engineered microbes

The engineered microbial cell disclosed herein can be produced by any genetic engineering method known to one of skill in the art without limitation.

Expression of an enzyme in a host cell can be accomplished by introducing into the host cells a nucleic acid comprising a nucleotide sequence encoding the enzyme under the control of regulatory elements that permit expression in the host cell. In some embodiments, the nucleic acid is an extrachromosomal plasmid. In other embodiments, the nucleic acid is a chromosomal integration vector that can integrate the nucleotide sequence into the chromosome of the host cell. Exemplary techniques of introduction of nucleic acids encoding these enzymes into the host cell include but are not limited to spheroplasting, electroporation, PEG 1000 mediated transformation, and lithium acetate or lithium chloride mediated transformation.

Overexpression of an enzyme in a host cell may be accomplished, for example, by providing multiple copies of a gene or by altering the promoter region to provide stronger expression. The copy number of an enzyme in a host cell may be altered by modifying the transcription of the gene that encodes the enzyme. This can be achieved for example by modifying the copy number of the nucleotide sequence encoding the enzyme (e.g., by using a higher or lower copy number expression vector comprising the nucleotide sequence, or by introducing additional copies of the nucleotide sequence into the genome of the host cell), or by increasing the strength of the promoter or operator to which the nucleotide sequence is operably linked.

In some embodiments, a nucleic acid used to genetically modify a host cell comprises one or more selectable markers, e.g., an antibiotic resistance marker, useful for the selection of transformed host cells and for placing selective pressure on the host cell to maintain the foreign DNA. an antibiotic resistance marker.

Inhibition of an enzyme activity in a host cell may be accomplished, for example, by deleting or disrupting the nucleotide sequence encoding the enzyme in the genome of the host cell. In some embodiments, the reduced activity can be achieved by introduction of gene disruption. The term "gene disruption" encompasses any genetic alteration that renders the encoded gene product inactive. The genetic alteration can be, for example, deletion or knockout of the entire gene, deletion of a regulatory sequence required for transcription or translation, deletion of a portion of the gene which results in a truncated gene product, or by any of various mutation strategies that inactivate the encoded gene product.

In certain embodiments, one particularly useful method of gene disruption may be complete gene deletion or knockout because it reduces or eliminates the occurrence of genetic reversions in the engineered microorganisms of the disclosure. The deletion or knockout of endogenous gene can be achieved by any gene editing systems known in the art, such as zinc finger nucleases (ZFNs), transcription activator-like effector nucleases (TALENs), or clustered regularly interspaced short palindromic repeat (CRISPR).

In other embodiments, the endogenous gene can also be knocked down by gene silencing, e.g., by using an oligonucleotide that binds to the gene coding for it, or to its mRNA, leading to a temporary change in expression. Exemplary gene silencing methods include, without limitation, RNA interference (RNAi), small interfering RNA (siRNA) or short hairpin RNA (shRNA), that are used to inactivate messenger RNA for a specific gene and effectively suppresses expression of the gene.

In other embodiments, inhibition of enzymatic activity can also be achieved by modifying or replacing endogenous genes to include a rare codon. For instance, when expressing a heterologous gene in *Escherichia coli*, the use of a rare codon that are infrequently employed by *E. coli* reduces the availability of homologous tRNAs, thereby decreasing the efficiency and level of protein expression. For a given species or cell, a person skilled in the art can readily identify a rare codon using various analytical tools, such as bioinformatics tools.

### Methods of Producing AAAs

In further aspects, methods or uses of producing AAAs using the engineered microbial cell as described herein are provided.

In some embodiments, provided herein is a method for producing aromatic amino acid, comprising culturing any of the aforementioned engineered microbial cell disclosed herein in a medium. In some embodiments, the method further comprises recovering aromatic amino acid from the culture. In some embodiments, the culturing continues for a time sufficient to produce AAA.

The method generally involves growing a host cell under suitable conditions in a suitable medium comprising a carbon source. Suitable conditions and suitable media for growing microorganisms are well known in the art. In some embodiments, the carbon source is a monosaccharide (simple sugar), a disaccharide, a polysaccharide, a non-fermentable carbon source, or one or more combinations thereof. Non-limiting examples of suitable monosaccharides include glucose, galactose, mannose, fructose, ribose, and combinations thereof. Non-limiting examples of suitable disaccharides include sucrose, lactose, maltose, trehalose, cellobiose, and combinations thereof. Non-limiting examples of suitable polysaccharides include starch, glycogen, cellulose, chitin, and combinations thereof. Non limiting examples of suitable non-fermentable carbon sources include acetate and glycerol. In some embodiments, the suitable medium is supplemented with one or more additional agents, such as, for example, an inducing compound (e.g., when one or more nucleotide sequences encoding a gene product are under the control of an inducible promoter), a repressing compound (e.g., when one or more nucleotide sequences encoding a gene product are under the control of a repressible promoter), or a selection agent (e.g., an antibiotic to select for microorganisms comprising the genetic modifications).

In some embodiments, the medium is suitable for *E. coli* culture.

In some embodiments, the medium may comprise LB medium supplemented with peptone, yeast extract, and NaCl. In some embodiments, the medium may comprise LB medium supplemented with 10g/l peptone, 5g/l yeast extract, and 10g/l NaCl.

The engineered microbial cell disclosed herein can produce an increased amount of AAA and/or a decreased amount of by-product compared to a host cell (e.g., wild-type or parent host cell) not comprising the genetic modification(s) of the engineered microbial cell disclosed herein, but is otherwise genetically identical.

In some embodiments, the increased amount is at least about 10% (e.g., at least about 20%, 30%, 40%, 50%, 60%, 70%, 80%, or 90%) or at least about 2-fold (e.g., about 3-fold, about 4-fold, about 5-fold, about 6-fold, about 7-fold, about 8-fold, about 9-fold, about 10-fold, about 15-fold, about 20-fold, about 30-fold, about 40-fold, about 50-fold, about 60-fold, about 70-fold, about 80-fold, about 90-fold, or about 100-fold), as measured under the same culture condition, for example, in grams per liter of cell culture, milligrams per gram of dry cell weight, on a per unit volume of cell culture basis, on a per unit dry cell weight basis, on a per unit volume of cell culture per unit time basis, or on a per unit dry cell weight per unit time basis.

In some embodiments, the decreased amount is at least about 10% (e.g., at least about 20%, 30%, 40%, 50%, 60%, 70%, 80%, or 90%) or at least about 2-fold (e.g., about 3-fold, about 4-fold, about 5-fold, about 6-fold, about 7-fold, about 8-fold, about 9-fold, about 10-fold, about 15-fold, about 20-fold, about 30-fold, about 40-fold, about 50-fold, about 60-fold, about 70-fold, about 80-fold, about 90-fold, or about 100-fold), as measured under the same culture condition, for example, in grams per liter of cell culture, milligrams per gram of dry cell weight, on a per unit volume of cell culture basis, on a per unit dry cell weight basis, on a per unit volume of cell culture per unit time basis, or on a per unit dry cell weight per unit time basis.

In some embodiments where the engineered microbial cell comprises an exogenous transketolase possessing a ratio of Km for G3P and/or F6P to Km for E4P and/or X5P that is less than 1:1 (e.g., less than 1:1.5, 1:2, 1:2.5, 1:3, 1:3.5, 1:4, 1:4.5 or 1:5) and a ratio of Km for G3P and/or S7P to Km for X5P and/or R5P that is less than 1:1 (e.g., less than 1:1.5, 1:2, 1:2.5, 1:3, 1:3.5, 1:4, 1:4.5 or 1:5), the engineered microbial cell produces an increased amount of AAA and/or a decreased amount of by-product compared to: (a) a host cell not comprising the exogenous transketolase but is otherwise genetically identical; or (b) a host cell comprising overexpression of endogenous transketolase but is otherwise genetically identical.

Also provided is use of any of the aforementioned engineered microbial cell disclosed herein in biosynthesis of aromatic amino acid.

### EXAMPLES

The examples provided below are for purposes of illustration only and are not intended to be limiting unless otherwise specified. Thus, the invention should in no way be construed as being limited to the following examples, but rather, should be construed to encompass any and all variations which become evident as a result of the teaching provided herein.

### EXAMPLE 1. Construction of tryptophan biosynthesis pathway vector

### 1. Acquisition of genes encoding tryptophan metabolic pathway

The major tryptophan synthesis pathway genes *trpA*, *trpB*, *trpC*, *trpD*, and *trpE* are all derived from *E.coli K-12* genome (NCBI Reference Sequence: NC_000913.3). Using the genome as a template, PCR amplification was carried out by using primers F1 and F2, and the resulting PCR product was named as *E.coli*-trp (6866bp).

The tryptophan synthesis pathway genes along with the tac promoter and RBS sequence were integrated into the backbone plasmid pUC19 (Addgene, #50005) using the ClonExpress MultiS one-step cloning kit (Vazyme, Jiangsu, China) to obtain the tryptophan pathway exogenous expression vector pUC-TrpEDCBA.

To increase the amount of tryptophan precursors, overexpression of *aroG* and *serA* was required. Using *E.coli K-12* genome as a template, PCR amplification was carried out using primers F2 and R2 to obtain a PCR product named *E.coli*-aroG (1061bp) and primers F3 and R3 to obtain a PCR product named *E.coli*-serA (1328bp).

The aroG and serA sequences along with tac promoter and RBS sequence were ligated together into the backbone plasmid pUC19 using the ClonExpress MultiS one-step cloning kit (Vazyme, Jiangsu, China). The ligation product was transformed into competent cell JM109 (commercial strain) using chemical transformation method, the positive clone was picked, the plasmid was extracted and sequenced, and the resulting correct recombinant plasmid was named as pTrp-GA vector.

### 2. Construction of tryptophan biosynthesis pathway vector pTrp-F

The gene expression vectors pUC-TrpEDCBA and pTrp-GA were used as templates for amplification using primers F4 and R4, and F5 and R5 to obtain the gene sequences with the tac promoter and RBS, respectively. pRGD-TcR (Addgene, #74110) was used as template for amplification using primers F6 and R6 to obtain the linearized vector.

The TrpEDBCA sequence with the tac promoter and the aroG and serA sequences with the tac promoter were ligated into the backbone plasmid pRGD-TcR using the ClonExpress MultiS one-step cloning kit (Vazyme, Jiangsu, China). The ligation product was transformed into JM109 competent cells by chemical transformation method, and the positive clones were picked and the plasmids were extracted for sequencing, and the resulting correct recombinant plasmids were named as pTrp-F vectors.

The resulting vector pTrp-F was transformed into wild-type strain M000, and the recombinant strain was named M000-Trp.

### EXAMPLE 2. Knockout of gnd gene

### 2.1 pSC101-gnd-KO vector construction

The upstream homology arm of *gnd* gene was amplified using primers gnd-HA1-F and gnd-HA1-R, and the downstream homology arm was amplified using primers gnd-HA2-F and gnd-HA2-R. Then overlapping PCR was performed with the upstream and downstream homology arms as templates using primers gnd-HA1-F and gnd-HA2-R, and the obtained fragment was named gnd-HA fragment.

The *pSC101-PBAD-sgRNA* (Huang, C., et al., Efficient long fragment editing technique enables large-scale and scarless bacterial genome engineering. Applied Microbiology and Biotechnology, 2020. 104(18): p. 7943-7956.) was used as a template and amplified using primers gnd-backbone-F and gnd-backbone-R to obtain the linearized vector fragment containing the corresponding sgRNA sequence.

The gnd-HA fragment and the linearized vector fragment were ligated by using ClonExpress MultiS one-step cloning kit (Vazyme, Jiangsu, China); and then transformed into competent cell JM109 using the chemical transformation method. The positive clones were picked, and the plasmids were extracted for sequencing. The resulting correct recombinant plasmid was named as pSC101-gnd-KO.

### 2.2 Knockout of gnd gene

The E.coli-K12 wild-type strain (M000, lab stored) was subjected to gene knockout by using λ-Red-mediated CRISPR-Cas9 method, and the *gnd* gene was deleted from the genome of M000 to generate the mutant strain M001.

The p15A-PBAD-Cas9-PT5-Redγβα vector (Huang, C., et al., 2020.) expressing λ-Red and CRISPR-Cas9 system was first transformed into competent cells of strain M000, which were then prepared as chemically competent cells. The vector *pSC101-gnd-KO* for *gnd* knockout was transformed into M000 competent cells containing the vector p15A-PBAD-Cas9-PT5-Redγβα, and gene editing was performed after single colonies were grown.

Single colonies were inoculated in LB and recovered for 2 hours, followed by addition of kanamycin and ampicillin at final concentrations of 50 µg/L and 100 µg/L respectively, as well as IPTG at a final concentration of 1 mM for 1 hour of cultivation , followed by addition of 1 M L-arabinose at a final concentration of 200 mM for 3 hours of cultivation ; and then 1 µl of the culture was coated onto LB agar plates containing Amp, Kan, and L-arabinose, and cultured at 30°C overnight. PCR was performed using the primers gnd-verification-F and gnd-verification-R to verify whether the gnd gene had been successfully knocked out. The knockout strain was named as strain M001.

### 2.3 Construction of fermentation strain M001-Trp

The tryptophan biosynthesis pathway vector pTrp-F was transformed into the competent cells of strain M001 to obtain the recombinant strain named M001-Trp and transformed into the competent cells of wild-type strain to obtain the control strain.

### 2.4 Growth status of recombinant strain M001-Trp

The bacterial solution of OD600=1 was inoculated at 1% in 200 ml of seed medium (LB medium supplemented with 10g/l peptone, 5g/l yeast extract, and 10g/l NaCl) containing a final concentration of 20 µl/mL of tetracycline, and when the OD600 reached 4-5, the seed solution was transferred to a 2L fermenter containing medium, and the growth of the strain was real-time monitored.

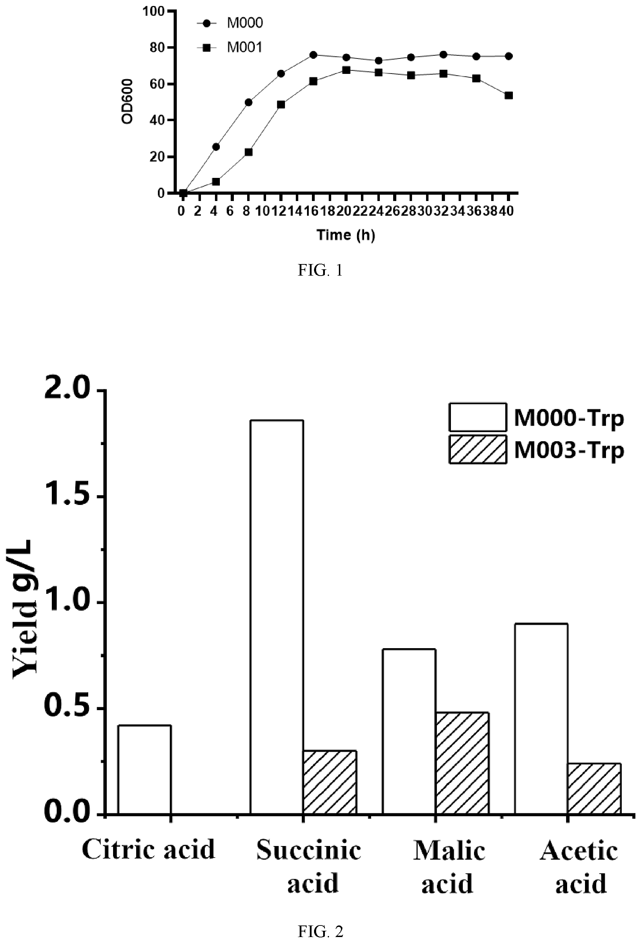

As shown in Figure 1, knockout of *gnd* gene affect the growth of the strain.

### EXAMPLE 3. Knockout of endogenous tktA

### 3.1 pSC101-tktA-KO vector construction

The upstream homology arm of endogenous *tktA* gene was amplified using primers tktA-HA1-F and tktA-HA1-R, and the downstream homology arm was amplified using primers tktA-HA2-F and tktA-HA2-R. Then, using the upstream and downstream homology arms as templates, overlapping PCR was carried out using primers tktA-HA1-F and tktA-HA2-R, and the obtained fragment was named tktA-HA fragment.

The pSC101-PBAD-sgRNA was used as a template and amplified using primers tktA-backbone-F and tktA-backbone-R to obtain the linearized vector fragment containing the corresponding sgRNA.

The tktA-HA fragment and the linearized vector fragment were ligated by using ClonExpress MultiS one-step cloning kit (Vazyme, Jiangsu, China), and transformed into the competent cell JM109 using chemical transformation method, the positive clones were picked, and the plasmids were extracted for sequencing. The resulting correct recombinant plasmid was named as pSC101-tktA-KO.

### 3.2 Knockout of endogenous tktA gene

Knockout genes of strain M001 were performed using the λ-Red-mediated CRISPR-Cas9 method to delete the *tktA* gene from the M001 genome and generate the mutant strain M002.

The p15A-PBAD-Cas9-PT5-Redγβα vector expressing λ-Red and CRISPR-Cas9 system were firstly transformed into the competent cells of M001, and the colonies were allowed to grow and inoculated to prepare them as chemically competent cells. The vector pSC101-tktA-KO for tktA knockout was transformed into M001 competent cells containing the vector p15A-PBAD-Cas9-PT5-Redγβα, and gene editing was performed after single colonies were grown.

Single colonies were inoculated in LB and recovered for 2 hours , followed by the addition of kanamycin and ampicillin at final concentrations of 50 µg/L and 100 µg/L respectively and IPTG at a final concentration of 1 mM for 1 hour of cultivation, followed by the addition of 1 M L-arabinose at a final concentration of 200 mM for 3 hours of cultivation, and 1 µl of the culture was coated onto LB agar plates containing Amp, Kan, and L-arabinose, and cultured at 30°C overnight. PCR was performed using the primers tktA-verification-F and tktA-verification-R to verify whether the *tktA* gene had been successfully knocked out. The knockout strain was named as strain M002.

### EXAMPLE 4. Overexpression of exogenous tkt4.1 Insertion of exogenous wild-type tkt gene

The mutant strain M003 was generated by inserting the exogenous *tkt* gene encoding a wild-type TKT (SEQ ID NO: 1) into strain M000 using a λ-Red-mediated CRISPR-Cas9 method.

First, the *tkt* fragment was obtained using primers tkt-F and tkt-R, with a synthetic gene vector containing the exogenous transketolase-encoding gene (SEQ ID NO: 1) as the template. Subsequently, using pSC101-tktA-KO as the template, the linearized plasmid was obtained by amplification using primers tkt-backbone-F and tkt-backbone-R. The *tkt* fragment and the linearized plasmid were then ligated using the ClonExpress MultiS One-Step Cloning Kit (Vazyme, Jiangsu, China) and transformed into competent cells JM109 via chemical transformation method. Positive clones were picked, and the plasmids were extracted for sequencing. The resulting correct recombinant plasmid was named as pSC101-tkt-In.

The vector of p15A-PBAD-Cas9-PT5-Redγβα expressing λ-Red as well as the CRISPR-Cas9 system was transformed into the competent cells of M000, and the colonies were allowed to grow and inoculated to prepare them as chemically competent cells. The vector pSC101-tkt-In for tkt insertion into the genome was transformed into M000 competent cells containing the vector p15A-PBAD-Cas9-PT5-Redγβα, and gene editing was carried out after single colonies were grown.

Single colonies were inoculated in LB and recovered for 2 hours, followed by the addition of kanamycin and ampicillin at final concentrations of 50 µg/L and 100 µg/L respectively and IPTG at a final concentration of 1 mM for 1 hour of cultivation , followed by the addition of 1 M L-arabinose at a final concentration of 200 mM for 3 hours of cultivation , and 1 µl of the culture was coated onto LB agar plates containing Amp, Kan, and L-arabinose, and cultured at 30°C overnight. PCR was performed using the primers tktA-verification-F and tktA-verification-R to verify whether the *tkt* gene had been successfully inserted into the genome. The recombinant strain was named as strain M003.

### 4.2 Construction of fermentation strains M000-Trp and M003-Trp

The tryptophan biosynthesis pathway vector pTrp-F was transformed into the competent cells of M000 and M003 strains to obtain the recombinant strains M000-Trp and M003-Trp respectively. The control strain was the wild-type strain containing the pTrp-F plasmid.

### 4.3 Tryptophan production of recombinant strains M000-Trp and M003-Trp

Tryptophan synthesis was carried out according to the following steps: the bacterial solution with OD600=1 was inoculated at 1% in 200 ml of seed medium containing a final concentration of 20 µl/mL of tetracycline, and when OD600 reached 4-5, the seed solution was transferred to a 2L fermenter containing medium, and the amount of tryptophan in the fermentation broth was measured after 40 hours of fermentation.

As shown in Figure 2, strain M000-Trp accumulated higher levels of organic acids during fermentation, including 0.42 g/L citric acid, 1.86 g/L succinic acid, 0.78 g/L malic acid, and 0.9 g/L acetic acid. Strain M003-Trp accumulated lower amounts of organic acids, with citric acid not detected, succinic acid at only 0.3 g/L, malic acid at only 0.48 g/L, and acetic acid at only 0.24 g/L.

### EXAMPLE 5. Comparison between exogenous wild-type tkt gene and tkt mutant

### 5.1 Construction of pSC101-tkt-R356L-In vector

The pSC101-tkt-In vector was used as a template for PCR amplification using primers R356L-F and R356L-R to obtain a fragment named tkt-R356L with correct size. The fragment was transformed into the competent cell JM109 using the chemical transformation method, and a single clone was picked out and the plasmid was extracted for sequencing, and the resulted correct recombinant plasmid was named as pSC101-tkt-R356L-In.

### 5.2 Insertion of exogenous tkt gene

Gene insertion was performed by inserting the exogenous wild-type *tkt* gene and *tkt* mutant (tkt-R356L, SEQ ID NO: 12) into strain M001 using the λ-Red-mediated CRISPR-Cas9 method to generate mutant strains M004 and M005 respectively.

The vector p15A-PBAD-Cas9-PT5-Redγβα expressing λ-Red and the CRISPR-Cas9 system was firstly transformed into the competent cells of M001, and the colonies were allowed to grow and inoculated to prepare them as chemically competent cells. The vectors pSC101-tktA-In and pSC101-tktA-R356L-In were transformed into M001 competent cells containing the vector p15A-PBAD-Cas9-PT5-Redγβα, and gene editing was carried out after single colonies were grown.

Single colonies were inoculated to LB for 2 hours of recovery, followed by the addition of kanamycin and ampicillin at final concentrations of 50 µg/L and 100 µg/L respectively and IPTG at a final concentration of 1 mM for 1 hour of cultivation , followed by the addition of 1 M L-arabinose at a final concentration of 200 mM for 3 hours of cultivation , and 1 µl of the culture was coated onto LB agar plates containing Amp, Kan, and L-arabinose, and cultured at 30°C overnight. PCR was performed using the validation primers tkt-verification-F and tkt-verification-R to verify whether the tkt gene had been successfully inserted into the genome. The recombinant strains were noted as: strain M004 and M005.

### 5.3 Construction of M004-Trp and M005-Trp fermentation strains

The tryptophan biosynthesis pathway vector pTrp-F was transformed into the competent cells of strains M004 and M005, respectively, to obtain recombinant strains named as M004-Trp and M005-Trp.

### 5.4 Tryptophan production by recombinant strains M004-Trp and M005-Trp

Tryptophan synthesis was carried out according to the following steps: the bacterial solution with OD600=1 was inoculated at 1% in 200 ml of seed medium containing a final concentration of 20 µl/mL of tetracycline inoculum, and then the seed solution was transferred to a 2L fermenter containing medium when OD600 reached 4-5, and fermentation was carried out for 40 hours.

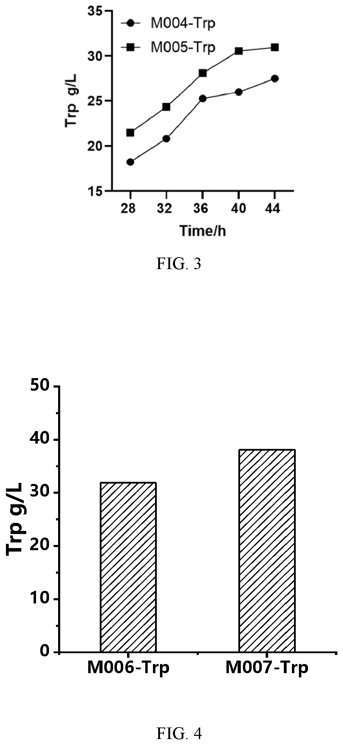

As shown in Figure 3, recombinant strain M004-Trp expressing wild-type tkt produced about 28 g/L of tryptophan, while strain M005-Trp expressing mutant tkt-R356L produced about 32 g/L of tryptophan. The results show that mutant tkt-R356L has higher tryptophan production potential compared to the wild-type tkt.

### EXAMPLE 6. Conversion of pyruvate to PEP

### 6.1 Construction of ptkt-wt and ptkt-wt-ppsA vectors expressing tkt and ppsA genes

The vector with p15A replicon and spectinomycin resistance was used as a template and PCR was performed using primers vector-F1 and vector-R1 to obtain a linearized vector.

Using ClonExpress MultiS one-step cloning kit (Vazyme, Jiangsu, China), the tkt-wt fragment (comprising the gene encoding exogenous transketolase (SEQ ID NO: 1)) was ligated with the linearized plasmid vector, transformed into the competent cell JM109 using chemical transformation method, positive clones were picked, and the plasmid was extracted for sequencing, and the resulting correct recombinant plasmid was named as ptktA-wt.

Using the ptkt-wt vector as a template, PCR was performed using primers vector-tkt-F1 and vector-tkt-R1 to obtain the linearized vector. Then, using ClonExpress MultiS one-step cloning kit (Vazyme, Jiangsu, China), the ppsA fragment was ligated with the linearized plasmid vector, transformed into the competent cell JM109 using chemical transformation method, the positive clones were picked, and the plasmid was extracted for sequencing, and the resulting correct recombinant plasmid was named as ptktA-wt-ppsA.

### 6.2 Construction of recombinant strains M006 and M007

The resulting vectors ptkt-wt and ptkt-wt-ppsA were transformed into M000 strains to obtain recombinant strains M006 and M007, respectively.

### 6.3 Construction of M006-Trp and M007-Trp fermentation strains

The tryptophan fermentation pathway vector pTrp-F was transformed into the competent cells of strains M006 and M007 to obtain recombinant strains named as M006-Trp and M007-Trp respectively.

### 6.4 Tryptophan production in recombinant strains M006-Trp and M007-Trp

Tryptophan synthesis was carried out according to the following steps: the bacterial solution with OD600=1 was inoculated at 1% in 200 ml of seed medium containing a final concentration of 20 µl/mL of tetracycline, and the seed solution was transferred to a 2L fermenter containing medium at OD600=4-5, and the amount of tryptophan in the fermentation broth was measured after 40 hours of fermentation.

As shown in Figure 4, overexpression of *ppsA* significantly increased tryptophan production from 31.9 g/L to 38.15 g/L. Therefore, the enhancement of tryptophan production could be achieved by converting pyruvate to phosphoenolpyruvate through overexpression of *ppsA* gene.

### EXAMPLE 7. Engineering nitrogen (N) cycle

### 7.1 Construction of knockout vector pSC101-gltBD-KO

The upstream homology arm of the *gltBD* gene was amplified using primers gltBD-HA1-F and gltBD-HA1-R, and the downstream homology arm was amplified using primers gltBD-HA2-F and gltBD-HA2-R. Then the upstream and downstream homology arms were used as templates, and overlapping PCR was carried out using primers gltBD-HA1-F and gltBD-HA2-R, and the resulting fragment was named as gltBD-HA fragment.

The pSC101-PBAD-sgRNA was used as a template and amplified using primers gltBD-sgRNA-F and gltBD-sgRNA-R. The fragment obtained was named gltBD-sgRNA. Linearized plasmid vectors were obtained by amplification using primers gltBD-backbone-F and gltBD-backbone-R.

Using ClonExpress MultiS one-step cloning kit (Vazyme, Jiangsu, China), the gltBD-HA fragment, gltBD-sgRNA fragment and linearized plasmid vector were ligated and transformed into the competent cell JM109 using the chemical transformation method, and the positive clones were picked and the plasmids were extracted. The resulting correct recombinant plasmid was named as pSC101-gltBD-KO.

### 7.2 Knockout of the gltBD gene

Gene knockout was performed in wild-type strain using the λ-Red-mediated CRISPR-Cas9 method to generate the mutant strain M014.

The p15A-PBAD-Cas9-PT5-Redγβα vector expressing λ-Red and CRISPR-Cas9 system was firstly transformed into the M000 strain competent cells, and the colonies were allowed to grow, and inoculated to prepare them into chemically competent cells. The vector pSC101-gltBD-KO for gltBD knockout was transformed into M000 strain containing vector p15A-PBAD-Cas9-PT5-Redγβα, and gene editing was performed after single colonies were grown.

The single colony was inoculated to LB liquid for recovery for 2 hours, followed by the addition of kanamycin at 50 µg/L, ampicillin at 100 µg/L, and IPTG at 1 mM for 1 hour of cultivation, followed by the addition of L-arabinose at a final concentration of 200 mM for 3 hours of cultivation. 1 µl of the culture was coated onto a low-salt LB agar plate containing Amp, Kan, and L-arabinose, and incubated at 30°C overnight. PCR was performed using the validation primers gltBD-verification-F and gltBD-verification-R to verify whether the *gltBD* gene had been successfully knocked out. The strain knocked out of gltBD was noted as M014.

### 7.3 M014-Trp fermentation strain construction

The tryptophan fermentation pathway vector pTrp-F was transformed into the competent cells of strain M014, and the recombinant strain was obtained and labeled as M014-Trp.

### 7.4 Tryptophan production by recombinant strain M014-Trp

Tryptophan synthesis was carried out according to the following steps: the bacterial solution with OD600=1 was inoculated at 1% in 200 ml of seed medium containing a final concentration of 20 µl/mL of tetracycline, and the seed solution was transferred to a 2L fermenter containing fresh medium at OD600=4-5, and the amount of tryptophan in the fermentation broth was determined after 40 hours of fermentation.

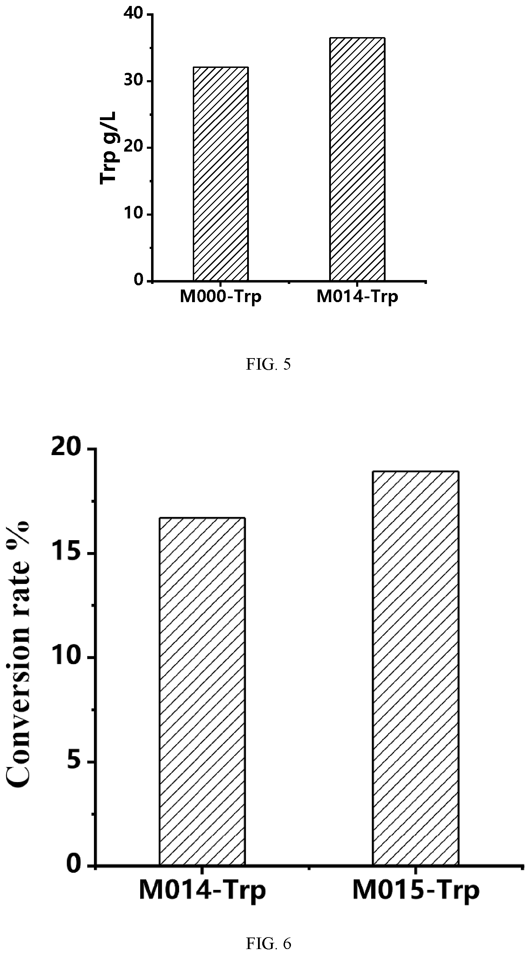

As shown in Figure 5, knockout of *gltBD* increased tryptophan production from 32.11g/L to 36.51g/L. Thus, modification on N cycle could facilitate the increase of tryptophan production.

### EXAMPLE 8. Enhancing nitrogen (N) cycle

### 8.1 Acquisition of glnA and gdhA genes

The *glnA* gene of *E. coli* was obtained from *E. coli. K-12* genome. Using the strain genome as a template, PCR amplification was performed using primer glnA-F1 and primer glnA-R1, and the resulting correct PCR product was named E.coli-glnA (1478bp).

The *gdhA* gene of *E. coli* was obtained from *E. coli. K-12* genome. Using the strain genome as a template, PCR amplification was performed using primer gdhA-F1 and primer gdhA-R1, and the resulting correct PCR product was named as E.coli-gdhA (1413bp).

Overlapping PCR was performed by using primers glnA-F1 and gdhA-R1, and the resulting fragment was named as glnA-gdhA (2870bp).

### 8.2 Construction of expression vector pglnA-gdhA

The vector with p15A replicon and spectinomycin resistance was used as a template and PCR was performed using primers vector-F1 and vector-R1 to obtain a linearized vector.

After that, by using ClonExpress MultiS one-step cloning kit (Vazyme, Jiangsu, China), the glnA-gdhA fragment was ligated with the linearized plasmid vector, and transformed into the competent cell JM109 using the chemical transformation method, and the positive clones were picked, and the plasmid was extracted for sequencing, and the resulting correct recombinant plasmid was named as pglnA-gdhA.

### 8.3 Construction of recombinant strain overexpressing glnA and gdhA genes

The resulting plasmid pglnA-gdhA was transformed into strain M014-Trp obtained in Example 7 by chemical transformation method to obtain a tryptophan-producing strain M015-Trp, in which *gltBD* was knocked out and *glnA* and *gdhA* were overexpressed.

### 8.4 Production of M014-Trp and M015-Trp strains

Tryptophan synthesis was carried out according to the following steps: the bacterial solution with OD600=1 was inoculated at 1% in 200 ml of seed medium containing a final concentration of 20 µl/mL of tetracycline, and when OD600=4-5 the seed solution was transferred to a 2L fermenter containing fresh medium, and the amount of tryptophan in the fermentation broth was determined after 36 hours of fermentation. And conversion rate was calculated according to the following formula: [amount of tryptophan produced] g/ [amount of glucose added] g x 100%.

As shown in Figure 6, the conversion rate for strain M014-Trp was 16.7% and the conversion rate for strain M015-Trp was 18.93%, indicating that combination of overexpression of *glnA* and *gdhA* and knockout of *gltBD* could improve the conversion rate.

### EXAMPLE 9. Combined modification of nitrogen cycle and tkt strategy

### 9.1 Construction of M016 strain

The mutant strain M016 was generated by knocking out *gnd* gene from strain M014 by using λ-Red-mediated CRISPR-Cas9 method.

The vector p15A-PBAD-Cas9-PT5-Redγβα (expressing λ-Red and CRISPR-Cas9 system) was first transformed into competent cells of M014, which were then prepared as chemically competent cells. The vector pSC101-gnd-KO for *gnd* knockout was transformed into M014 competent cells containing the vector p15A-PBAD-Cas9-PT5-Redγβα, and gene editing was carried out after single colonies were grown.

Single colonies were inoculated in LB for 2 hours of recovery, followed by the addition of kanamycin and ampicillin at final concentrations of 50 µg/L and 100 µg/L respectively, as well as IPTG at a final concentration of 1 mM for 1 hour of cultivation , followed by addition of 1M L-arabinose at a final concentration of 200 mM for 3 hours of cultivation ; and 1 µl of the culture was coated onto LB agar plates containing Amp, Kan, and L-arabinose, and cultured at 30°C overnight. PCR was performed using the primers gnd-verification-F and gnd-verification-R to verify whether the gnd gene had been successfully knocked out. The recombinant strain was named as strain M016.

### 9.2 Construction of M017 strain

Firstly, the vector of p15A-PBAD-Cas9-PT5-Redγβα (expressing λ-Red and CRISPR-Cas9 system) was transformed into the competent cells of M016, which were then prepared as chemically competent cells. The vector pSC101-tktA-R356L-In for tkt insertion was transformed into M016 competent cells containing the vector p15A-PBAD-Cas9-PT5-Redγβα, and gene editing was carried out after single colonies were grown.

Single colonies were inoculated in LB for 2 hours of recovery, followed by the addition of kanamycin and ampicillin at final concentrations of 50 µg/L and 100 µg/L respectively, as well as IPTG at a final concentration of 1 mM for 1 hour of cultivation, followed by addition of 1M L-arabinose at a final concentration of 200 mM for 3 hours of cultivation; and then 1 µl of the culture was coated onto LB agar plates containing Amp, Kan, and L-arabinose, and cultured at 30°C overnight. PCR was performed using the primers tkt-verification-F and tkt-verification-R to verify whether the tkt gene had been successfully inserted into the genome. The recombinant strain was noted as: strain M017.

### 9.3 Construction of M017-Trp fermentation strain

The tryptophan fermentation pathway vector pTrp-F was transformed into the competent cells of strain M017 to obtain the recombinant strain labeled as M017-Trp.

### 9.4 Tryptophan production by recombinant strain M017-Trp

Tryptophan synthesis was carried out using tryptophan-producing strains M000-Trp and M017-Trp according to the following steps: the bacterial solution with OD600=1 was inoculated at 1% in 200 ml of seed medium containing a final concentration of 20 µl/mL of tetracycline, and when OD600=4-5, the seed solution was transferred to the 2L fermenter containing medium, and the amount of tryptophan in the fermentation broth was determined after 40 hours of fermentation and the L-tryptophan production per OD was calculated.

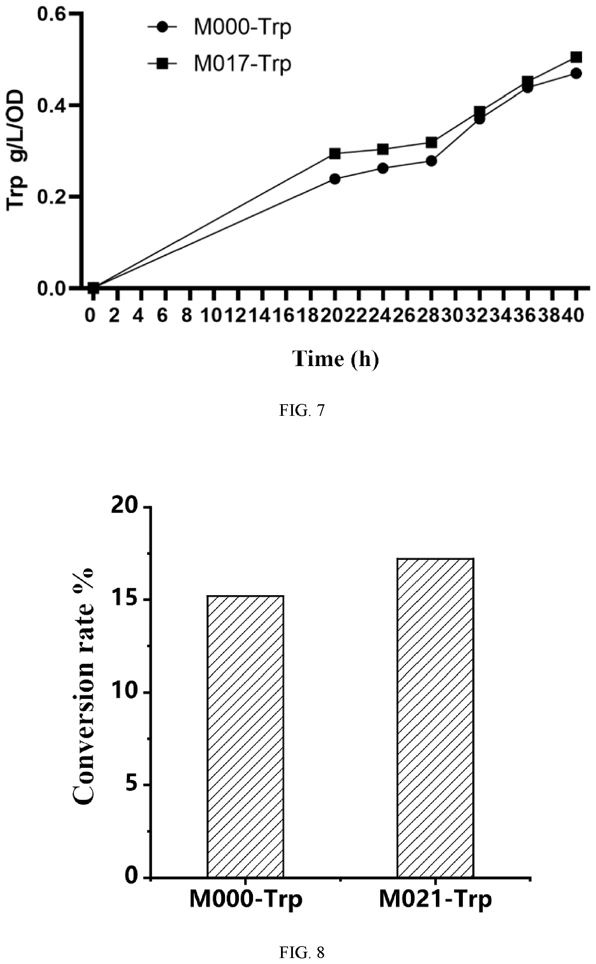

As shown in Figure 7, after 40 hours of fermentation, the Trp yield for control strain M000-Trp was 0.48 g/L/OD, and for strain M017-Trp was 0.51 g/L/OD, indicating that the combined modifications of knockout of *gltBD*, knockout of *gnd* and insertion of exogenous tktA-R356L is effective in enhancing production of tryptophan.

### EXAMPLE 10. Combined Modifications of Carbon Cycle, Nitrogen Cycle, and tkt Strategy

### 10.1 Construction of Strain M018

The mutant strain M018 was generated by inserting the gene *tkt* encoding an exogenous wild-type transketolase (TKT), and the phosphoenolpyruvate synthase gene *ppsA* into strain M002 using a λ-Red-mediated CRISPR-Cas9 method.

First, the upstream homology arm of the endogenous *tktA* gene was amplified using primers tkt-ppsA-HA1-F and tkt-ppsA-HA1-R. The downstream homology arm was amplified using primers tkt-ppsA-HA2-F and tkt-ppsA-HA2-R. The exogenous *tkt* gene fragment was amplified using primers tkt-F and tkt-R, and the *ppsA* gene fragment was amplified using primers ppsA-F and ppsA-R. Subsequently, using the upstream homology arm, downstream homology arm, exogenous *tkt* gene fragment, and *ppsA* gene fragment as templates, overlap PCR was performed with primers tkt-ppsA-HA1-F and tkt-ppsA-HA2-R. The resulting fragment was named as the tkt-ppsA-HA fragment. The pSC101-PBAD-sgRNA was used as a template and amplified with primers tkt-ppsA-backbone-F and tkt-ppsA-backbone-R to obtain the linearized vector fragment containing the corresponding sgRNA. The tkt-ppsA-HA fragment and the linearized vector fragment were ligated using the ClonExpress MultiS One-Step Cloning Kit (Vazyme, Jiangsu, China) and transformed into competent cells JM109 via chemical transformation method. Positive clones were picked, and plasmids were extracted for sequencing. The resulting correct recombinant plasmid was named pSC101-tkt-ppsA-In.

The p15A-PBAD-Cas9-PT5-Redγβα vector, expressing the λ-Red and CRISPR-Cas9 systems, was transformed into competent cells M002 and the colonies were allowed to grow and inoculated to prepare them as chemically competent cells. The vector pSC101-tkt-ppsA-In, for inserting tkt-ppsA into the genome, was then transformed into M002 competent cells containing the vector p15A-PBAD-Cas9-PT5-Redγβα, and gene editing was performed after single colonies were grown.

Single colonies were inoculated into LB medium and allowed to recover for 2 hours. Then, kanamycin and ampicillin were added to final concentrations of 50 µg/L and 100 µg/L, respectively, along with IPTG to a final concentration of 1 mM. After 1 hour of cultivation, 1 M L-arabinose was added to a final concentration of 200 mM, and cultivation continued for 3 hours. 1µl culture was spread onto LB agar plates containing Amp, Kan, and L-arabinose, and cultured at 30°C overnight. PCR was performed using primers tkt-ppsA-verification-F and tkt-ppsA-verification-R to verify whether the *tkt* and *ppsA* genes had been successfully inserted into the genome. The recombinant strain was named as strain M018.

### 10.2 Construction of Strain M019

Mutant strain M019 was generated by knocking out the *gltBD* gene from strain M018 using a λ-Red-mediated CRISPR-Cas9 method. Vector p15A-PBAD-Cas9-PT5-Redγβα (expressing λ-Red and CRISPR-Cas9 systems) was transformed into competent cells M018, which were then prepared as chemically competent cells. The vector pSC101-gltBD-KO, designed for *gltBD* knockout, was transformed into M018 competent cells containing p15A-PBAD-Cas9-PT5-Redγβα, and gene editing was carried out after single colonies were grown.

Single colonies were inoculated into LB medium and recovered for 2 hours. Kanamycin and ampicillin were added to final concentrations of 50 µg/L and 100 µg/L, respectively, along with IPTG to 1 mM. After 1 hour of cultivation, 1M L-arabinose was added to a final concentration of 200 mM, and cultivation continued for 3 hours. 1µl of culture was spread onto LB agar plates containing Amp, Kan, and L-arabinose, and cultured overnight at 30°C. PCR was performed using primers gltBD-verification-F and gltBD-verification-R to verify whether the *gltBD* gene had been successfully knocked out. The recombinant strain was designated as strain M019.

### 10.3 Construction of Strain M020

Mutant strain M020 was generated by knocking out the *talA-tktB* genes from strain M019 using a λ-Red-mediated CRISPR-Cas9 method.

First, the upstream homology arm of the endogenous *tktB-talA* gene was amplified using primers tktB-talA-HA1-F and tktB-talA-HA1-R. The downstream homology arm was amplified using primers tktB-talA-HA2-F and tktB-talA-HA2-R. Overlap PCR was then performed using the upstream and downstream homology arms as templates with primers tktB-talA-HA1-F and tktB-talA-HA2-R. The resulting fragment was named tktB-talA-HA fragment. The pSC101-PBAD-sgRNA was used as a template and amplified using primers tktB-talA-backbone-F and tktB-talA-backbone-R to obtain the linearized vector fragment containing the corresponding sgRNA. The tktB-talA-HA fragment and the linearized vector fragment were ligated using the ClonExpress MultiS One-Step Cloning Kit (Vazyme, Jiangsu, China) and transformed into competent cells JM109 via chemical transformation method. Positive clones were picked, and plasmids were extracted for sequencing. The resulting correct recombinant plasmid was named pSC101-tktB-talA-KO.

The vector p15A-PBAD-Cas9-PT5-Redγβα (expressing the λ-Red and CRISPR-Cas9 systems) was transformed into competent cells M019, which were then made chemically competent cells. The vector pSC101-tktB-talA-KO, designed for *talA-tktB* genes knockout, was transformed into these M019 competent cells containing the plasmid p15A-PBAD-Cas9-PT5-Redγβα, and gene editing was carried out after single colonies were grown.

Single colonies were inoculated into LB and allowed to recover for 2 hours. Then, kanamycin and ampicillin were added to final concentrations of 50 µg/L and 100 µg/L, respectively, along with IPTG to a final concentration of 1 mM. After 1 hour of cultivation, 1M L-arabinose was added to a final concentration of 200 mM, and cultivation continued for 3 hours. 1µl of culture was spread on LB agar plates with Amp, Kan, and L-arabinose, and cultured overnight at 30°C. PCR was performed using primers talA-tktB-verification-F and talA-tktB-verification-R to verify whether the *talA-tktB* genes had been successfully knockout. The recombinant strain was designated as strain M020.

### 10.4 Construction of Strain M021

Mutant strain M021 was generated by knocking out the *talB* gene from strain M020 using a λ-Red-mediated CRISPR-Cas9 method.

First, the upstream homology arm of the endogenous *talB* gene was amplified with primers talB-HA1-F and talB-HA1-R. The downstream homology arm was amplified with primers talB-HA2-F and talB-HA2-R. Using the upstream and downstream homology arm as templates, overlap PCR was performed with primers talB-HA1-F and talB-HA2-R. The resulting fragment was designated as the talB-HA fragment. The pSC101-PBAD-sgRNA was used as a template and amplified with primers talB-backbone-F and talB-backbone-R to obtain the linearized vector fragment containing the corresponding sgRNA. The talB-HA fragment and the linearized vector fragment were ligated using ClonExpress MultiS One-Step Cloning Kit (Vazyme, Jiangsu, China) and transformed into competent cells JM109 via chemical transformation method. Positive clones were picked, and plasmids were extracted for sequencing. The resulting correct recombinant plasmid was named pSC101-talB-KO.

The vector p15A-PBAD-Cas9-PT5-Redγβα (expressing the λ-Red and CRISPR-Cas9 systems) was transformed into competent cells M020, which were then made chemically competent cells. The vector pSC101-talB-KO, designed for *talB* gene konockout*,* was transformed into M020 competent cells containing the vector p15A-PBAD-Cas9-PT5-Redγβα, and gene editing was carried out after single colonies were grown.

Single colonies were inoculated into LB medium and allowed to recover for 2 hours. Then, kanamycin and ampicillin were added to final concentrations of 50 µg/L and 100 µg/L, respectively, along with IPTG to a final concentration of 1 mM. After 1 hour of cultivation, 1M L-arabinose was added to a final concentration of 200 mM, and cultivation continued for 3 hours. 1µl culture was spread onto LB agar plates containing Amp, Kan, and L-arabinose, and incubated at 30°C overnight. PCR was performed using primers talB-verification-F and talB-verification-R to verify whether the *talB* gene had been successful knocked out. The recombinant strain was designated as strain M021.

### 10.5 Construction of M021-Trp Fermentation Strain

The tryptophan fermentation pathway vector pTrp-F was transformed into competent cells of strain M021 to obtain the recombinant strain designated M021-Trp.

### 10.6 Tryptophan Production by Recombinant Strain M021-Trp

Tryptophan synthesis was performed using tryptophan-producing strains M000-Trp and M021-Trp following these steps: a bacterial solution with OD600 = 1 were inoculated at 1% ratio into 200 mL of seed medium containing tetracycline at a final concentration 20 µL/mL. When OD600= 4-5, the seed culture was transferred to a 2 L fermenter. After 40 hours of fermentation, the amount of tryptophan in the fermentation broth was measured, and the L-tryptophan yield per OD was calculated.

As shown in Figure 8, after 40 hours of fermentation, the Trp conversion rate of control strain M000-Trp was 15.2%, while the Trp conversion rate of strain M021-Trp was 17.2%. This indicates that the combined modifications-overexpression of *ppsA*, knockout of *gltBD*, knockout of *gnd*, knockout of endogenous *tkt* and introduction of exogenous *tkt*, and knockout of *tal*-effectively enhanced tryptophan production.

### EXAMPLE 11. Combined Modifications of tkt Strategy and attenuated TCA Cycle

### 11.1 Construction of Strain M022

Mutant strain M022 was generated by performing rare codon replacement on the *gltA* gene in strain M021 using a λ-Red-mediated CRISPR-Cas9 method.

First, the upstream homology arm of the endogenous *gltA* gene was amplified using primers gltA-HA1-F and gltA-HA1-R. The downstream homology arm was amplified using primers gltA-HA2-F and gltA-HA2-R. The *gltA* gene fragment with rare codon replacement was amplified using primers gltA-F and gltA-R. Then, using the upstream and downstream homology arm, and *gltA* gene fragment as templates, overlap PCR was performed with primers gltA-HA1-F and gltA-HA2-R. The resulting fragment was named the gltA-HA fragment. The pSC101-PBAD-sgRNA was used as a template and amplified with primers gltA-backbone-F and gltA-backbone-R to obtain the linearized vector fragment containing the corresponding sgRNA. The gltA-HA fragment and the linearized vector fragment were ligated using the ClonExpress MultiS One-Step Cloning Kit (Vazyme, Jiangsu, China) and transformed into competent cells JM109 via chemical transformation method. Positive clones were picked, and plasmids were extracted for sequencing. The resulting correct recombinant plasmid was named pSC101-gltA-In.

The vector p15A-PBAD-Cas9-PT5-Redγβα (expressing the λ-Red and CRISPR-Cas9 systems) was transformed into competent cells M021, which were then prepared as chemically competent cells. The vector pSC101-gltA-In, used for replacing *gltA*, was transformed into M021 competent cells containing the vector p15A-PBAD-Cas9-PT5-Redγβα. After single colonies grew, gene editing was performed.

Single colonies were inoculated into LB medium and allowed to recover for 2 hours. Then, kanamycin and ampicillin were added to final concentrations of 50 µg/L and 100 µg/L, respectively, along with IPTG to a final concentration of 1 mM. After 1 hour of cultivation, 1M L-arabinose was added to a final concentration of 200 mM, and cultivation continued for 3 hours. 1µl culture was spread onto LB agar plates containing Amp, Kan, and L-arabinose, and incubated overnight at 30°C. PCR was performed using primers gltA-verification-F and gltA-verification-R, followed by sequencing, to verify whether the *gltA* gene had been successful replaced. The recombinant strain was designated as strain M022.

### 11.2 Construction of Fermentation Strain M022-Trp

The tryptophan fermentation pathway vector pTrp-F was transformed into competent cells of strain M022 to obtain the recombinant strain designated M022-Trp.

### 11.3 Tryptophan Production by Recombinant Strain M022-Trp

Tryptophan synthesis was carried out using tryptophan-producing strains M021-Trp and M022-Trp following these steps: a bacterial solution with OD600=1 was inoculated at 1% into 200 mL of seed medium containing tetracycline at a final concentration of 20 µl/mL. When OD600 = 4-5, the seed culture was transferred into a 2 L fermenter. After 40 hours of fermentation, the amount of tryptophan in the fermentation broth was measured.

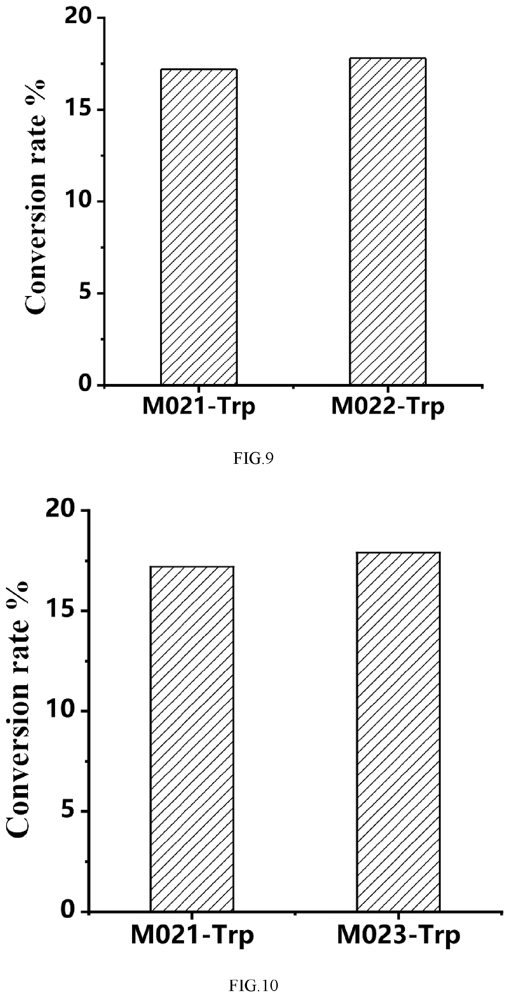

As shown in Figure 9, after 40 hours of fermentation, the Trp conversion rate of control strain M021-Trp was 17.2%, while the Trp conversion rate of strain M022-Trp was 17.8%. This indicates that the combined modification of the tkt strategy and attenuated TCA cycle effectively enhances tryptophan production.

### EXAMPLE 12. Knockout of the zwf Gene

### 12.1 Construction of Strain M023

Mutant strain M023 was generated by knocking out *zwf* gene from strain M021 using a λ-Red-mediated CRISPR-Cas9 method.

First, the upstream homology arm of the endogenous *zwf* gene was amplified using primers zwf-HA1-F and zwf-HA1-R. The downstream homology arm was amplified using primers zwf-HA2-F and zwf-HA2-R. Then, using the upstream and downstream homology arms as templates, overlap PCR was performed with primers zwf-HA1-F and zwf-HA2-R. The resulting fragment was named the *zwf-HA* fragment. The pSC101-PBAD-sgRNA was used as a template and amplified with primers zwf-backbone-F and zwf-backbone-R to obtain the linearized vector fragment containing the corresponding sgRNA. The *zwf-HA* fragment and the linearized vector fragment were ligated using the ClonExpress MultiS One-Step Cloning Kit (Vazyme, Jiangsu, China) and transformed into competent cells JM109 via chemical transformation method. Positive clones were picked, and plasmids were extracted for sequencing. The resulting correct recombinant plasmid was named pSC101-zwf-KO.

The vector p15A-PBAD-Cas9-PT5-Redγβα (expressing the λ-Red and CRISPR-Cas9 systems) was transformed into competent cells M021, which were then prepared as chemically competent cells. The vector pSC101-zwf-KO, designed for *zwf* knockout, was transformed into M021 competent cells containing vector p15A-PBAD-Cas9-PT5-Redγβα. After single colonies grew, gene editing was performed.

Single colonies were inoculated into LB medium and allowed to recover for 2 hours. Then, kanamycin and ampicillin were added to final concentrations of 50 µg/L and 100 µg/L, respectively, along with IPTG to a final concentration of 1 mM. After 1 hour of cultivation, 1M L-arabinose was added to a final concentration of 200 Mm, and cultivation continued for 3 hours. 1µl culture was spread onto LB agar plates containing Amp, Kan, and L-arabinose, and incubated overnight at 30°C. PCR was performed using primers zwf-verification-F and zwf-verification-R to verify whether the *zwf* gene had been successful knocked out. The recombinant strain was designated as strain M023.

### 12.2 Construction of Fermentation Strain M023-Trp

The tryptophan fermentation pathway vector pTrp-F was transformed into competent cells of strain M023 to obtain the recombinant strain labeled M023-Trp.

### 12.3 Tryptophan Production by Recombinant Strain M023-Trp

Tryptophan synthesis was carried out using tryptophan-producing strains M021-Trp and M023-Trp following these steps: a bacterial solution with OD600=1 was inoculated at 1% into 200 mL of seed medium containing tetracycline at a final concentration of 20 µl/mL. When OD600 =4-5, the seed culture was transferred into a 2 L fermenter. After 40 hours of fermentation, the amount of tryptophan in the fermentation broth was measured.

As shown in Figure 10, after 40 hours of fermentation, the Trp conversion rate of control strain M021-Trp was 17.2%, while the Trp conversion rate of strain M023-Trp was 17.9%. This indicates that knockout of *zwf* effectively enhances tryptophan production.

### SEQUENCES

SEQ ID NO: 1 (TKT from *Scheffersomyces stipitis*) 
MSSVDQKAISTIRLLAVDAVAAANSGHPGAPLGLAPAAHAVFKKMRFNPKDTKWINRDR
FVLSNGHACALLYSMLVLYGYDLTVEDLKKFRQLGSKTPGHPENTDVPGAEVTTGPLGQ
GICNGVGIALAQAQFAATYNKPDFPISDSYTYVFLGDGCLMEGVSSEASSLAGHLQLGN
LIAFWDDNKISIDGSTEVAFTEDVIARYKSYGWHIVEVSDADTDITAIAAAIDEAKKVTNK
PTLVRLTTTIGFGSLAQGTHGVHGAPLKADDIKQLKTKWGFNPEESFAVPAEVTASYNEH
VAENQKIQQQWNELFAAYKQKYPELGAELQRRLDGKLPENWDKALPVYTPADAAVATR
KLSEIVLSKIIPEVPEIIGGSADLTPSNLTKAKGTVDFQPAATGLGDYSGRYIRYGVREHAM
GAIMNGIAAFGANYKNYGGTFLNFVSYAAGAVRLSALSEFPITWVATHDSIGLGEDGPTH
QPIETLAHFRATPNISVWRPADGNETSAAYKSAIESTHTPHILALTRQNLPQLEGSSIEKAS
KGGYTLVQQDKADIIIVATGSEVSLAVDALKVLEGQGIKAGVVSLPDQLTFDKQSEEYKL
SVLPDGVPILSVEVMSTFGWSKYSHQQFGLNRFGASGKAPEIFKLFEFTPEGVAERAAKT
VAFYKGKDVVSPLRSAF
SEQ ID NO: 2 (TKT from *Leishmania mexicana*) 
MASIEKVANCIRCLAADIVQGGKSGHPGTPMGMAPMSAVLWTEVMKYNSQDPDWVDR
DRFVMSNGHGCALQYALLHMAGYNLTMDDLKGFRQDGSRTPGHPERFVTPGVEVTTG
 
PLGQGIANAVGLAIAEAHLAATFNRPGYNIVDHYTYVYCGDGCLMEGVCQEALSLAGH
LALEKLIVIYDSNYISIDGSTSLSFTEQCHQKYVAMGFHVIEVKNGDTDYEGLRKALAEA
KATKGKPKMIVQTTTIGFGSSKQGTEKVHGAPLGEEDIANIKAKFGRDPQKKYDVDDDV
RAVFRMHIDKCSAEQKAWEELLAKYTAAFPAEGAAFVAQMRGELPSGWEAKLPTNSSAI
ATRKASENCLAVLFPAIPALMGGSADLTPSNLTRPASANLVDFSSSSKEGRYIRFGVREHA
MCAILNGLDAHDGIIPFGGTFLNFIGYALGAVRLAAISHHRVIYVATHDSIGVGEDGPTHQ
PVELVAALRAMPNLQVIRPSDQTETSGAWAVALSSIHTPTVLCLSRQNTEPQSGSSIEGVR
HGAYSVVDVPDLQLVIVASGSEVSLAVDAAKALSGELRVRVVSMPCQELFDAQPDTYRQ
AVLPAGVPVVSVEAYVSFGWEKYSHAHVGMSGFGASAPAGVLYKKFGITVEEVVRTGR
ELAKRFPDGTAPLKNSSFSKM
SEQ ID NO: 3 (TKT from *Debaryomyces hansenii*) 
MSQDLDQLAISTIRLLSVDSVDKAQSGHPGAPLGLAPAAHAVWQKMKFNPKDTNWINR
DRFILSNGHACALLYSLLVLYGYDLTVEDLKQFRQLNSKTPGHPEALDVPGCEVTTGPLG
QGISNAVGLAMAQKQFGATYNKPDHKISDSYTYAFLGDGCLMEGVASETCSLAGHLQL
NNLIAFWDDNHISIDGNTNVAFTEDVMLRFKAYGWHLIEIAEGDTDLDAIAKAIDEAKK
VEDKPTLIRLTTTIGFGSLAQGTHGVHGAPLKADDIKQLKESMGFNPEETFVIPKEVTDY
LKKHVEENQKVQEDWKKLFESYKQKYPELGAELQRRLDGKLPENWSDALPTYSPSDSA
VATRKLSETVLTKILPVIPEIIGGSADLTGSNLTRAAGSVDFQPPATGLGDYSGRYIRYGVR
EHGMGAIMNGIAAFGANYKNYGGTFLNFVSYGAGAVRLSALSHHPVIWVATHDSIGLGE
DGPTHQPIETLAHLRAIPNLSVWRPADGNETSAAYLQAIESTSTPSIIALTRQNLPQLEGSSI
EKAAKGGYTLVKQDKPDVIIVSSGSEVSISADAAKKLESEGFKAGVVSMPDFLTFDKQPL
DYRLSVLPDGVPIMSVEVMSSFGWSKYSHEQFGLNRFGASGKGPEIYKFFEFTPEGVAD
RAAKTIQFYKGKPLLSPLNRAF
SEQ ID NO: 4 (TKT from *Suhomyces tanzawaensis*) 
MSQDLDQLTINTIRLLSVDAVAAANSGHPGAPLGLAPAAHAVWKQMKFNPRDTKWINR
DRFILSNGHACALLYSLLVLYGYDLEVEDLKQFRQLNSKTPGHPEALDVPGVEVTTGPL
GQGISNAVGLALAQTQFAATYNKSDVQISNSYTYSFLGDGCLMEGVASEASSLAGHLQL
GNLIAFWDDNRISIDGSTEVAFTENVVERYKAYGWHVLEVPHGDTDLEGIANAIEEAKK
VTDKPTLIRLTTTIGFGSLAQGTHGVHGSPLKADDIKQLKEKWGFNPEENFAIPAEVKNY
LSEIVKKNQEGHEEWKKTFDQYKQKYPELGAEVQRRLDGKLPEGWEKNLPTYTPQDK
AVATRKLSEIVLQSVYDAVPELIGGSADLTPSNLTRTTSAVDFQPPSTGLGDYSGRYIRYGV
REHGMGAIMNGIAAFGANYKNYGGTFLNFVSYAAGAVRLSALSHHPVTWVATHDSIGL
 
GEDGPTHQPIETLAHFRAIPNVSVWRPADGNETSAAYKSAISSTSTPHIIALTRQNLPQLEG
SSIENASKGGYTLVKQDNPDVIIVASGSEVSIAVDASKKLAEEGFKAGVVSLPDFFTFDNQ
SQSYRLSVLPDGVPIISVEVMSTFGWSKYSHEQFGLNRFGASGKGPELYKFFEFTPEGIAD
RAAKTIQFYKGKPVLSPLNRAF
SEQ ID NO: 5 (TKT from *Candida subhashii*) 
MSSIDQKAVSTIRLLAVDAVAAANSGHPGAPLGLAPAAHVLWSNMKFNPRDTKWINRDR
FVLSNGHACALLYSLLVLFKYDLTVEDLKQFRQLGSKTPGHPEVLDVPGVEVTTGPLGQ
GIANGVGLAIAQKQFAATYNKPDITISDSYTYVFMGDGCLMEGVSGEASSLAGHLKLNN
LIAFWDDNKISIDGDTEVSFDEDVVARYRAYGWNIVEVADANTDLQAISNAIEEAKKCQ
DKPTLIRLVTTIGFGSLAAGTHGVHGAPLKADDIKQLKAKWGFNPEKSFAIPEEVEQFYD
SKIAENQKVYRQWQATFESYKQKYPKEGAELQRRLDGKLPEGWKEALPRFTPQDKAVA
TRKLSEGVIEALSPIIPEFIGGSADLTPSNLTRATGAVDFQPPSSGLGDYSGRYIRYGVREHG
MGAIMNGIAAFGANYKNYGGTFLNFVSYAAGAVRLSALSHHPITWVATHDSIGLGEDGP
THQPIETLAHFRAMPNVSVWRPADGNETSAAYLSAIESTSHPTIIALTRQNLPQLEGSSIEN
ASKGGYTLIKQDKPEVIIVASGSEVSISVEAAKRLESEGVKANVVSIPDFFTFDKQSLDYK
LSVLPDGVPIMSVEVMSTYGWSKYSHEQFGLNRFGASGKAPEIYKYFEFTAEGVAGRAA
KTIEFYKGKSVFSPLNTAF
SEQ ID NO:6 (TKT from *Candida tenuis*) 
MSTDQLAVNTIRLLAVDTVSKANSGHPGAPLGLAPAAHVVFQNMKFNPKDTKWINRDR
FVLSNGHACALLYTMLFLYGYDYTVEDLKSFRQLHSKTPGHPESTDCPGVEVTTGPLGQ
GICNAVGLALAQKQFAATYNKPNYTLSDNFTYCFLGDGCLMEGVSSEASSLAGHLQLGN
LIAFWDDNQISIDGSTEVAFTEDVIARYKAYAWHILEVEDGNSDLAGIAKAIEEAKKVTD
KPTLIRIKTTIGYGSLAQGTHGVHGAPLKPDDVKQLKSKFGFDPEEFFAVPKEVTESYQK
HVESNLKVQKEWEATFAAYKKEYPELGAEVQRRLDGKLPEGWEKALPSFTPADKSVAT
RKLSEGVINALSDILPEFIGGSADLTPSNLTKATGSVDFQPPSTGLGDYSGKYIRYGVREH
GMGAIMNGIAAFGANYKNYGGTFLNFVSYAAGAVRLSALSGHPITWVATHDSIGLGEDG
PTHQPIETLAHFRALPNCSVWRPADGNEVSAAYKSAVSSTSTPHILALSRQNLPQLEGSSI
EKASKGGYVLKEASKPDVILVASGSEVEIAVNAAKLLEAEGKKVSVVSIPDFLTFDKQSD
EYRLSVLPDGAPVVSIEVMSPFGWSKYSHEQFGLSRFGASGKAEDVYKYLEFTPEGVAE
RASKTITFYKGKEVLSPLTRAF
SEQ ID NO: 7 (TKT from *Hyphopichia burtonii*) MSELEQKAINTIRLLAVDAVAAANSGHPGAPLGMAPAAHAVWQTMKFNPKNPKWINRD 
RFVLSNGHACALLYSLLVLYGYDLSIEDLKQFRQLGSKTPGHPEALDVPGVEVTTGPLGQ
GISNAVGIALAQKQFAATYNKPDFQISDSNIFVFLGDGCLQEGVASEASSLAGHLKLNNII
AFWDDNHISIDGNTNVAFTEDVLGRYKAYGWNILEVENGNTDVDGITKAIEAAKKSDLP
TIIRLPTTIGFGSLAEGTHGVHGAPLKADDIKQLKTKFGFNPEESFVIPKEVTELYNKHVSE
NQQVENDWNKLFAAYKEKYPTEGAEVERRLSGKLPEGWEKSLPVYTPNDKPVATRKLS
EIVLSNVVPVVPEIIGGSADLTGSNLTRTTDMVDFQHPDTKLGDYSGRYIRYGVREHGMG
AIMNGIAAFGANYKNYGGTFLNFVSYAAGAVRLSALSHHPVTWVATHDSIGLGEDGPTH
QPIETLAHLRAIPNLSVWRPADGNETSAAYKSAIESTSTPHVIALTRQNLPQLEGSSIEVAS
KGGYTLIKQDKPDVIIAASGSEVAIAVDASKKLSEEGIKANVVSIPDFLTFDKQDQSYQLS
VLPDGVPILSVEVMSTFGWSKYSHEQFGLNRFGASGKGPAIYEYFGFTPEGVADKAAKT
VQFYKGKDVLSPLNKAF
SEQ ID NO: 8 (TKT from *Spathaspora passalidarum*) 
MTSATIDQKAVSTIRLLAVDAVAAANSGHPGAPLGLAPAAHVLWKQMKFNPRDTKWVN
RDRFVLSNGHACALLYSLLVLFKFDLHVSDLKQFRQLGSKTPGHPEVLDVPGVEVTTGP
LGQGIANAVGLAIAQKQFAATYNKPDITISDNYTYAFLGDGCLMEGVSGEASSLAGHLKL
NNLIAFWDDNKISIDGSTEVSFDEDVIARYRAYGWNIVEVEDADDNLEAIANAIVEAKKS
KDKPTLIKLVTIIGYGSLAQGTHGVHGSPLKADDIKQLKTKWGFNPEESFAIPQEVSDYY
SSHVQENIKVYNDWHATFKAYKEKYPKEGAEIQRRLDGKLPEGWKDALPRFTPADKAV
ATRKLSEGVISALSPIIPEFIGGSADLTPSNLTKVAGSIDFQPPSSGLGDYAGRYIRYGVREH
GMGAIMNGIAAFGANYKNYGGTFLNFVSYAAGAVRLSALSHHPVTWVATHDSIGLGED
GPTHQPIETLAHFRAIPNLSVWRPADGNETSAAYLSAVEATSHPTIIALTRQNLPQLEGSSI
EAASKGGYTLIKNDKPDVIIVASGSEVAISVDASKKLAEEGIKANVVSLPDFFTFDKQSFD
YKLSVLPDGVPIMSVEVMSTFGWSKYSHEQFGLNRFGASGKAPELYKYFEFTPEGVAGR
AAKTVQFYKGKTVYSPLNTAF
SEQ ID NO: 9 (TKT from *Candida intermedia*) 
MSDIDQLAISTIRLLAVDAVAAANSGHPGAPLGLAPAAHAVWKEMKFNPKNPKWINRDR
FVLSNGHACALLYALLHLYGFDLSIDDLKSFRQLNSKTPGHPEATDVPGVEVTTGPLGQG
ISNAVGLAIAQKQFAATYNKDDLTVSDSYVYTFLGDGCLMEGVSSEASSLAGHLQLNNLI
AFWDDNQISIDGNTNVAFTEDVIARYKAYGWHTIEIPHGDTDLEAVAAAIKEAKSVTDKP
TLVRLTTTIGFGSLAQGTHGVHGAPLKADDIKQLKEKFGFDPSKTFNVPQEVYDYYSKL
AKKNQEVEAEWDNTVAEYQKKYPEQGAELKRRLDGKLPEGWESALPSYTPEDKPLATR
KLSEMLLTKVLPVLPEVIGGSADLTGSNLTRAPDMVDFQPPSTKLGDWSGRYIRYGVRE
HGMGAIMNGIAGFGGNYKNYGGTFLNFVSYAAGAVRLSALSHLPVTWVATHDSIGLGE
 
DGPTHQPIETLAHFRATPNLSVWRPADGNEVSAAYKSAFESTSVPHIIALTRQNLPHLEGS
SLEKASKGGYTLVKADKPDVIIVSSGSEVSISVDAAKKLAGEGIQASVVSLPDFLTFDKQP
QDYRLSVLPDGVPIMSVEVMSSFGWAKYSHEQFGLNRFGASGKAEDLYKFFEFTPEGVA
ERASKTVQFYKGKEVVSPLNRAF
SEQ ID NO: 10 (TKT from *Pichia sorbitophila*) 
MGKDIDQLAISTIRTLSVDAVCKANSGHPGAPLGLAPAAHAVWKRMRFHPKDTKWINR
DRFVLSNGHACALLYSMLVLYGFDLSIDDMKQFRQVGSKTPGHPEALDVPGVEVTTGPL
GQGISNAVGLALAQKQFSATYNKPDFPICDSHVYVFLGDGCLMEGVSSESSSLAGHLQL
NNLIAFWDDNKISIDGDTNVAFTEDVMLRYKAYGWNIMEIEKGDSDLDGVSRAIDEAKK
STNKPTLIRLTTTIGYGSLEQGTHGVHGAPLKPDDQKQLKAKNGFDPEQYFVIPPEVTEY
YNEHVQENLKQYSEWQQLFNAYKQKYPELGAEVQRRLDGKLPADWSKILPSYKPSDKA
LATRKLSEMVLSKVLPAIPEVIGGSADLTGSNLTRITDSVDFQPPSTGLGDYTGRYIRYGV
REHGMGAIMNGIAAFGANYKNYGGTFLNFVSYGAGAVRLAAISHHPVTWVATHDSIGL
GEDGPTHQPVETLAHLRAIPNMSVWRPADGNEVSAAYLSAIESTSTPHVLALSRQNLPQL
EGSSIEKAAKGGYTLVSHEKPDIILVSSGSEVSIAVEAAKSLTEKGFKANVVSLVDFLTFDK
QPMDYKLSVLPDGVPIMSFEVLSTFGWSKYAHEQFGLNTFGMSGKAPEIYNAFEFTPQG
VADRAAKTVQFYKGKPILSPLNTAF
SEQ ID NO: 11 (TKT from *Pichia sorbitophila*) 
MGKDIDQLAISTIRILSVDAVCKANSGHPGAPLGLAPAAHAVWKKMRFHPKDTKWINRD
RFVLSNGHACALLYSMLVLYGFDLSIDDMKQFRQVGSKTPGHPEALDVPGVEVTTGPLG
QGISNAVGLALAQKQFAATYNKPDFPICDSHVYVFLGDGCLMEGVSSEASSLAGHLQLN
NLIAFWDDNKISIDGDTNVAFTEDVLLRYKAYGWNVMEIEKGDSDLDAVSKAVDEAKKS
TNKPTLIRLTTTIGYGSLQQGTHGVHGAPLKPDDLKQLKEKNGFDPEKFFVIPPEVTQYY
NEHVQENLKQYNEWQNLFNNYKQKYPELGAEVERRLEGKLPEDWSKVLPSYKPSDKP
LATRKLSEMVLSKVLPAIPEVIGGSADLTGSNLTRVADSVDFQAPSTGLGDYTGRYIRYGV
REHGMGAIMNGIAAFGANYKNYGGTFLNFVSYAAGAVRLAAISHHPVIWVATHDSIGLG
EDGPTHQPVETLAHLRAIPNLSVWRPADGNEVSAAYLNAIESTSTPHVLALSRQNLPQLE
GSSIEKAAKGGYTLVSHEKPDIILVSSGSEVSISVDAAKSLSEKGFKANVVSLVDFLTFDK
QPMDYKLSVLPDGVPIMSFEVLSTFGWSKYAHEQFGLNTFGMSGKAPEIYKAFEFTPEG
VADRAAKTIQFYKGKPILSPLNTAF
SEQ ID NO: 12 (TKT-R356L) MSSVDQKAISTIRLLAVDAVAAANSGHPGAPLGLAPAAHAVFKKMRFNPKDTKWINRDR 
FVLSNGHACALLYSMLVLYGYDLTVEDLKKFRQLGSKTPGHPENTDVPGAEVTTGPLGQ
GICNGVGIALAQAQFAATYNKPDFPISDSYTYVFLGDGCLMEGVSSEASSLAGHLQLGN
LIAFWDDNKISIDGSTEVAFTEDVIARYKSYGWHIVEVSDADTDITAIAAAIDEAKKVTNK
PTLVRLTTTIGFGSLAQGTHGVHGAPLKADDIKQLKTKWGFNPEESFAVPAEVTASYNEH
VAENQKIQQQWNELFAAYKQKYPELGAELQRRLDGKLPENWDKALPVYTPADAAVATL
KLSEIVLSKIIPEVPEIIGGSADLTPSNLTKAKGTVDFQPAATGLGDYSGRYIRYGVREHAM
GAIMNGIAAFGANYKNYGGTFLNFVSYAAGAVRLSALSEFPITWVATHDSIGLGEDGPTH
QPIETLAHFRATPNISVWRPADGNETSAAYKSAIESTHTPHILALTRQNLPQLEGSSIEKAS
KGGYTLVQQDKADIIIVATGSEVSLAVDALKVLEGQGIKAGVVSLPDQLTFDKQSEEYKL
SVLPDGVPILSVEVMSTFGWSKYSHQQFGLNRFGASGKAPEIFKLFEFTPEGVAERAAKT
VAFYKGKDVVSPLRSAF
SEQ ID NO: 13 (F1)
   ggcgtatcacgaggccctttgtgtgtcgacactcattaaaattag
SEQ ID NO: 14 (R1)
   cgttatcccctgattctgtgatgcaaacacaaaaaccgactctc
SEQ ID NO: 15 (F2)
   cagcgatacctttgccatTTATTAAACCttacccgcgacg
SEQ ID NO: 16 (R2)
   atgaattatcagaacgacgatttacg
SEQ ID NO: 17 (F3)
   AAatggcaaaggtatcgctg
SEQ ID NO: 18 (R3)
   ATCCTCTAGAGCAGCAACG
SEQ ID NO: 19 (F4)
   TCGACACTCATTAAAATTAGTCGCT
SEQ ID NO: 20 (R4)
   catgagaattatcactagtgaatcatcaTTGACAATTAATCATCGGCTCGTATAA
SEQ ID NO: 21 (F5)
   acgttaagggattttggtcatgTTGACAATTAATCATCGGCTCGTATAA
SEQ ID NO: 22 (R5)
   CTAATTTTAATGAGTGTCGAATCCTCTAGAGCAGCAACG
SEQ ID NO: 23 (F6)
   tgatgattcactagtgataattctcatg
SEQ ID NO: 24 (R6)
   catgaccaaaatcccttaacgt
SEQ ID NO: 25 (gnd-HA1-F)
   AAACAAATAGGGGTTCCGCGTGGAGTGGCGATGATGAC
SEQ ID NO: 26 (gnd-HA1-R)
   CACACCTGACAGGAGTATGTATCTGATTTAACCAACAATAAAATTGAGG
SEQ ID NO: 27 (gnd-HA2-F)
   TACATACTCCTGTCAGGTGTG
SEQ ID NO: 28 (gnd-HA2-R)
   TCTCCTTACGCATCTGTGCATACGGTTGTTGATTGGTGC
SEQ ID NO: 29 (gnd-backbone-F)
   GTCGTAATAGGCAACCGCTGGTTTTAGAGCTAGAAATAGCAAGTTAAAATAAG
SEQ ID NO: 30 (gnd-backbone-R)
   CGCGGAACCCCTATTTGTTT
SEQ ID NO: 31 (gnd-verification-F)
   CCGTTGATTTGATGACCATAACC
SEQ ID NO: 32 (gnd-verification-R)
   ACCTTCCTTAATAAAACAAAAATTCC
SEQ ID NO: 33 (tktA-HA1-F)
   aaacaaataggggttccgcggatgaacctgaattaaccg
SEQ ID NO: 34 (tktA-HA1-R) 
gtttcatcttcggggatcacTTGACAATTAATCATCGGCTCGTATAATGTGTGGAATTGTGAGCGG
ATAACAATTTCACACAGGAA
SEQ ID NO: 35 (tktA-HA2-F)
   tcgaaagactgggcctttcgttttatctgttgtttgtcggtgaacgctctcctgagtaggacaaatcgaacaatccttttgtgataaatg
SEQ ID NO: 36 (tktA-HA2-R)
   actagtattatacctaggactgagctagctgtcaattgcgtcaatctgatccttc
SEQ ID NO: 37 (tktA-backbone-F)
   cagtcctaggtataatactagtaaacgtgtgcgcaacgacatGTTTTAGAGCTAGAAATAGCAAG
SEQ ID NO: 38 (tktA-backbone-R)
   cgcggaacccctatttgttt
SEQ ID NO: 39 (tkt-F) 
GCGGATAACAATTTCACACAGGAAACAGCTATGACCATGATTACGGATTCACTGGAAT
TCCTTgaattcattaaagaggagaaaggtaca
SEQ ID NO: 40 (tkt-R) GCCCGCTGCGCAGCGCCTTTTGAcgtgctagaggcatcaaataaaacgaaaggctcagtcgaaagactgggcctttc gttttatctgttg
SEQ ID NO: 41 (tkt-backbone-F) 
tgtacctttctcctctttaatgaattcAAGGAATTCCAGTGAATCCGTAATCATGGTCATAGCTGTTTCCT
GTGTGAAATTGTTATCCGC
SEQ ID NO: 42 (tkt-backbone-F) 
caacagataaaacgaaaggcccagtctttcgactgagcctttcgttttatttgatgcctctagcacgTCAAAAGGCGCTGCGC
AGCGGGC
SEQ ID NO: 43 (tkt-verification-F)
   tctcgatgatcctttgtcgc
SEQ ID NO: 44 (tkt-verification-R)
   ccttcactttgcaagatgatgg
SEQ ID NO: 45 (R356L-F)
   CGCTGACGCTGCAGAACCTGCCGCAGTTGGAAGGCTCGAGCATT
SEQ ID NO: 46 (R356L-R)
   GCAGGTTCTGCAGCGTCAGCGCCAGAATATGCGGGGTATG
SEQ ID NO: 47 (gltBD-HA1-F)
   aaacaaataggggttccgcgcaaaaacagtagcatgaaacgtc
SEQ ID NO: 48 (gltBD-HA1-R)
   ggtgttaacaagtaattgagacggggatttatcgtacaacatatcgtgaac
SEQ ID NO: 49 (gltBD-HA2-F)
   ccgtctcaattacttgttaacacc
SEQ ID NO: 50 (gltBD-HA2-R)
   tctccttacgcatctgtgctggggtacatgcaccagg
SEQ ID NO: 51 (gltBD-sgRNA-F)
   gcacagatgcgtaaggaga
SEQ ID NO: 52 (gltBD-sgRNA-R)
   ccgtggacgtatttcagcagatggagaaacagtagagagagttgc
SEQ ID NO: 53 (gltBD-backbone-F)
   ctgctgaaatacgtccacggGTTTTAGAGCTAGAAATAGCAAGTTAAAATAAG
SEQ ID NO: 54 (gltBD-backbone-R)
   cgcggaacccctatttgttt
SEQ ID NO: 55 (glnA-F1) 
AAACAGCTATGACCATGATTACGGATTCACTGGAATTCCTTgaattcattaaagaggagaaaggtaca
atgtccgctgaacacgtac
SEQ ID NO: 56 (glnA-R1)
   ttagacgctgtagtacagctc
SEQ ID NO: 57 (gdhA-F1)
   gagctgtactacagcgtctaattaatatataagggttttatatctatggatcagaca
SEQ ID NO: 58 (gdhA-R1)
   gttttatttgatgcctctagcacgttaaatcacaccctgcgcc
SEQ ID NO: 59 (tkt-ppsA-HA1-F)
   AAACAAATAGGGGTTCCGCGGCGCTAACTTAAGGGTTGTGG
SEQ ID NO: 60 (tkt-ppsA-HA1-R) 
AGCTGTTTCCTGTGTGAAATTGTTATCCGCTCACAATTCCACACATTATACGAGCCGAT
GATTAATTGTCAATCTGCTGTCGGTTAGCTC
SEQ ID NO: 61 (tkt-ppsA-HA2-F) 
GTGAACGCTCTCCTGAGTAGGACAAATCCGCCGCCCTAGACCTAGGGGATATATTCCG
CTTCCTCGCTCACCGAATGTATCCGCTCACTT
SEQ ID NO: 62 (tkt-ppsA-HA2-R)
   TCTCCTTACGCATCTGTGCGCGGCGAGTAGATTAGCA
SEQ ID NO: 63 (tkt-F) 
CACACAGGAAACAGCTATGACCATGATTACGGATTCACTGGAATTCCTTGAATTCATT
AAAGAGGAGAAAGGTACAATGAGCAGCGTCGA
SEQ ID NO: 64 (tkt-R)
   TGTACCAATGAATTCTCATCAAAAGGCGCTGCGC
SEQ ID NO: 65 (ppsA-F)
   TGAGAATTCATTGGTACAATGTCCAACAATGGCTCG
SEQ ID NO: 66 (ppsA-R) 
ACAACAGATAAAACGAAAGGCCCAGTCTTTCGACTGAGCCTTTCGTTTTATTTGATGC
CTCTAGCACGTTATTTCTTCAGTTCAGCCAGGC
SEQ ID NO: 67 (tkt-ppsA-backbone-F)
   GCACAGATGCGTAAGGAGA
SEQ ID NO: 68 (tkt-ppsA-backbone-R)
   CGCGGAACCCCTATTTGTTT
SEQ ID NO: 69 (tkt-ppsA-verification-F)
   GCGCTCAAAGAGCTTCTTTG
SEQ ID NO: 70 (tkt-ppsA-verification-R)
   GCGCTGGTAACGAGAAAAGA
SEQ ID NO: 71 (tktB-talA-HA1-F)
   AAACAAATAGGGGTTCCGCGTACCTCGGGCGGTATATTTG
SEQ ID NO: 72 (tktB-talA-HA1-R)
   GCACCTTTCACTCCCAGCTGCCGTCTAACTCGTTCATAG
SEQ ID NO: 73 (tktB-talA-HA2-F)
   GCTGGGAGTGAAAGGTGC
SEQ ID NO: 74 (tktB-talA-HA2-R) 
GACGGCACTAGTATTATACCTAGGACTGAGCTAGCTGTCAACTTGATCCATTGCGTCG
TG
SEQ ID NO: 75 (tktB-talA-backbone-F) 
GGTATAATACTAGTGCCGTCGCCCATAAACACATGTTTTAGAGCTAGAAATAGCAAGT
TA
SEQ ID NO: 76 (tktB-talA-backbone-R)
   CGCGGAACCCCTATTTGTTT
SEQ ID NO: 77 (tktB-talA-verification-F)
   GCCCTTGCCTTCCATCAC
SEQ ID NO: 78 (tktB-talA-verification-R)
   GGTTAACCCAACGCCGAC
SEQ ID NO: 79 (talB-HA1-F)
   AAACAAATAGGGGTTCCGCGGCAAGACTGGTACACAATGAC
SEQ ID NO: 80 (talB-HA1-R)
   GGTGTACTGACGAAGGGAG
SEQ ID NO: 81 (talB-HA2-F)
   CTCCCTTCGTCAGTACACCATGATCGGCGATCTGCTG
SEQ ID NO: 82 (talB-HA2-R) 
CGCTCAGACTAGTATTATACCTAGGACTGAGCTAGCTGTCAACGAAAGGATCGCAGTT
GG
SEQ ID NO: 83 (talB-backbone-F) 
GGTATAATACTAGTCTGAGCGAAGGAGAACAGCAGTTTAGAGCTAGAAATAGCAAG
TTA
SEQ ID NO: 84 (talB-backbone-R)
   CGCGGAACCCCTATTTGTTT
SEQ ID NO: 85 (talB-verification-F)
   CAACCCACATCCAAAAGACG
SEQ ID NO: 86 (talB-verification-R)
   AGCGTCTCTTTAATAGACTTCGG
SEQ ID NO: 87 (gltA-F) 
GTTGAACTGGATGTGCTGAAAGGCACGCTGGGTCAAGATGTTATAGATATACGTACTC
TCGGTTCAAAAGGTGTGTTCACCTTTGACCC
SEQ ID NO: 88 (gltA-R) 
TATATCGCTTTTAAAGTCGCGTTTTTCATATCCTGTATACAGCTGACGCGGACGGGCTA
TCTTCATACCGTCA
SEQ ID NO: 89 (gltA-HA1-F)
   AAACAAATAGGGGTTCCGCGCGGCAGTATAGGCTGTTCAC
SEQ ID NO: 90 (gltA-HA1-R)
   TTTCAGCACATCCAGTTCAAC
SEQ ID NO: 91 (gltA-HA2-F)
   GCGACTTTAAAAGCGATATAAAGCGTTAATGGTTGATTGCTAAG
SEQ ID NO: 92 (gltA-HA2-R)
   TGCACTAGTATTATACCTAGGACTGAGCTAGCTGTCAACAGAACCTTCATCAGCAGC
SEQ ID NO: 93 (gltA-backbone-F) 
CAGTCCTAGGTATAATACTAGTGCAGTCATGTGTGGTATTACGTTTTAGAGCTAGAAAT
AGCAAGTTA
SEQ ID NO: 94 (gltA-backbone-R)
   CGCGGAACCCCTATTTGTTT
SEQ ID NO: 95 (gltA-verification-F)
   GGATGGTCTGTAGGTCCAG
SEQ ID NO: 96 (gltA-verification-R)
   GTTGTCGTGACTTGTCCAAG
SEQ ID NO: 97 (zwf-HA1-F)
   AAACAAATAGGGGTTCCGCGGCATGTCGTTATAGGAGGTG
SEQ ID NO: 98 (zwf-HA1-R)
   ATGGTCGTTCCTGGAATGAG
SEQ ID NO: 99 (zwf-HA2-F)
   CTCATTCCAGGAACGACCATAGCAATTTACGACGCGCAAG
SEQ ID NO: 100 (zwf-HA2-R)
   ACTAGTATTATACCTAGGACTGAGCTAGCTGTCAATCAAATGTTCCAGCTGAGAC
SEQ ID NO: 101 (zwf-backbone-F) 
CAGTCCTAGGTATAATACTAGTACGCAGGTAGAATGGCACACGTTTTAGAGCTAGAAA
TAGCAAGTTA
SEQ ID NO: 102 (zwf-backbone-R)
   CGCGGAACCCCTATTTGTTT
SEQ ID NO: 103 (zwf-verification-F)
   GCAGGGCTTTACGAATGATTTC
SEQ ID NO: 104 (zwf-verification-R)
   TGCGACAGAAACGATTCACC

## Claims

1. An engineered microbial cell that produces aromatic amino acid, comprising:
(a) heterologous expression of exogenous transketolase or a variant thereof, wherein the exogenous transketolase or a variant thereof possesses a higher catalytic efficiency (e.g., lower Km and/or higher kcat) or transketolase activity for glyceraldehyde-3-phosphate (G3P) and/or fructose-6-phosphate (F6P) than for erythrose-4-phosphate (E4P) and/or xylulose 5-phosphate (X5P); and
(b) blocked or reduced flux through pentose phosphate pathway.

2. The engineered microbial cell of claim 1, wherein the exogenous transketolase or a variant thereof possesses a ratio of Km for G3P or F6P to Km for E4P or X5P that is less than 1:1, e.g., less than 1:1.5, 1:2, 1:2.5, 1:3, 1:3.5, 1:4, 1:4.5 or 1:5.

3. The engineered microbial cell of claim 1 or 2, wherein the exogenous transketolase or a variant thereof further possesses a lower Km for glyceraldehyde-3-phosphate (G3P) or sedoheptulose-7-phosphate (S7P) than for xylulose 5-phosphate (X5P) or ribose-5-phosphate (R5P); e.g., possesses a ratio of Km for G3P or S7P to Km for X5P or R5P that is less than 1:1, e.g., less than 1:1.5, 1:2, 1:2.5, 1:3, 1:3.5, 1:4, 1:4.5 or 1:5.

4. The engineered microbial cell of any one of claims 1 to 3, wherein the exogenous transketolase is derived from *Leishmania mexicana*, *Scheffersomyces stipites*, *Suhomyces tanzawaensis*, *Candida subhashii*, *Candida tenuis*, *Hyphopichia burtonii*, *Spathaspora passalidarum*, *Candida intermedia*, or *Pichia sorbitophila*; preferably, the exogenous transketolase comprises the amino acid sequence of anyone of SEQ ID NOs: 1-12 or functionally equivalent variants thereof.

5. The engineered microbial cell of any one of claims 1 to 4, comprising blocked or reduced oxidative phase of pentose phosphate pathway.

6. The engineered microbial cell of claim 5, comprising reduced activity of 6-phosphogluconate dehydrogenase and/or glucose-6-phosphate dehydrogenase, e.g., achieved by deleting, knocking out or knocking down *gnd* gene and/or *zwf* gene, or by modifying or replacing the endogenous *gnd* gene and/or *zwf* gene to contain a rare codon.

7. The engineered microbial cell of any one of claims 1 to 6, further comprising enhanced flux through nitrogen assimilation pathway, e.g., comprising increased activity of glutamate dehydrogenase and/or glutamine synthetase, reduced activity of glutamate synthase, or any combination thereof.

8. The engineered microbial cell of claim 7, wherein *gltBD* gene is deleted, knocked out, or knocked down.

9. The engineered microbial cell of claim 7 or 8, comprising overexpression of *gdhA* and/or *glnA* genes.

10. The engineered microbial cell of any one of claims 1 to 9, comprising: (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene or *zwf* gene; optionally further comprising (iii) deletion, knockout, or knockdown of endogenous *gltBD* gene.

11. The engineered microbial cell of any one of claims 1 to 10, further comprising reduced activity of endogenous transketolase, e.g., achieved by deleting, knocking out or knocking down endogenous *tktA* gene and/or *tktB* gene, or by modifying or replacing the endogenous *tktA* gene and/or *tktB* gene to contain a rare codon.

12. The engineered microbial cell of any one of claims 1 to 11, further comprising reduced activity of endogenous transaldolase, e.g., achieved by deleting, knocking out or knocking down endogenous *talA* gene and/or *talB* gene, or by modifying or replacing the endogenous *talA* gene and/or *talB* gene to contain a rare codon.

13. The engineered microbial cell of any one of claims 1 to 12, further comprising blocked or attenuated flux through TCA cycle.

14. The engineered microbial cell of claim 13, comprising reduced activity of pyruvate dehydrogenase and/or citrate synthase, e.g., achieved by deleting, knocking out or knocking down *lpd*, *aceF*, *aceE* gene or *gltA* gene, or by modifying or replacing *lpd*, *aceF*, *aceE* gene or *gltA* gene to contain a rare codon, preferably, achieved by modifying or replacing the endogenous *gltA* gene to contain a rare codon.

15. The engineered microbial cell of any one of claims 1 to 14, further comprising blocked or reduced oxaloacetate biosynthesis pathway.

16. The engineered microbial cell of claim 15, comprising reduced activity of phosphoenolpyruvate carboxylase, e.g., achieved by deleting, knocking out or knocking down *ppc*, or by modifying or replacing the endogenous *ppc* gene to contain a rare codon.

17. The engineered microbial cell of any one of claims 1 to 16, wherein the naturally occurring phosphotransferase (PTS) transport system has been disabled.

18. The engineered microbial cell of claim 17, comprising reduced activity of one or more PTS system protein(s), e.g., achieved by deleting, knocking out or knocking down one or more gene(s) selected from *ptsH*, *ptsI*, *crr*, *ptsG*, or any combination thereof, or by modifying or replacing the endogenous *ptsH*, *ptsI*, *crr*, *ptsG* gene, or any combination thereof to contain a rare codon.

19. The engineered microbial cell of claim 17 or 18, further comprising increased activity of at least one non-PTS sugar transporter, e.g., overexpression of endogenous non-PTS sugar transporter or heterologous expression of exogenous non-PTS sugar transporter, such as glucose facilitator (Glf) of *Zymomonas mobilis*.

20. The engineered microbial cell of claim 19, further comprising increased activity of glucokinase, e.g., overexpression of endogenous *glk* gene or heterologous expression of exogenous *glk* gene, e.g., from *Zymomonas mobilis*.

21. The engineered microbial cell of any one of claims 1 to 20, further comprising blocked or reduced pathways for production of acetic acid and/or lactic acid.

22. The engineered microbial cell of claim 21, comprising reduced activity of one or more enzymes selected from phosphate acetyltransferase, pyruvate dehydrogenase, lactate dehydrogenase, or any combination thereof; e.g., achieved by deleting, knocking out or knocking down one or more gene(s) selected from *pta*, *poxB*, *ldhA*, or any combination thereof, or by modifying or replacing *pta*, *poxB*, *ldhA* gene, or any combination thereof to contain a rare codon.

23. The engineered microbial cell of any one of claims 1 to 22, further comprising an enhanced phosphoenolpyruvate (PEP) biosynthesis pathway.

24. The engineered microbial cell of claim 23, comprising increased activity of phosphoenolpyruvate synthase to enhance conversion from pyruvate to PEP, e.g., achieved by overexpression of endogenous *ppsA* or heterologous expression of exogenous *ppsA* gene.

25. The engineered microbial cell of claim 23, comprising increased activity of pyruvate carboxylase and PEP carboxykinase to enhance concersion from pyruvic acid to PEP, e.g., achieved by combined overexpression of both endogenous or exogenous *pyc* gene and endogenous or exogenous *pck* genes, e.g., achieved by overexpression of endogenous *pyc* and *pck* genes, or heterologous expression of exogenous *pyc* and *pck* genes, or achieved by overexpression of endogenous *pck* gene and heterologous expression of exogenous *pyc* gene (e.g., from *Cytobacillus firmus*).

26. The engineered microbial cell of any one of claims 1 to 25, comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and
(ii) deletion, knockout, or knockdown of endogenous *gnd* gene and/or *zwf* gene;
optionally the engineered microbial cell further comprises any one of the following:
(iii) deletion, knockout, or knockdown of endogenous *gltBD* gene;
(iv) deletion, knockout, or knockdown of endogenous *tktA* gene and/or *tktB* gene;
(v) deletion, knockout, or knockdown of endogenous *talA* gene and/or *talB* gene;
(vi) modification or replacement of endogenous *gltA* gene to contain a rare codon;
(vii) deletion, knockout, or knockdown of endogenous *ppc* gene;
(viii) deletion, knockout, or knockdown of endogenous *ptsH*, *ptsI*, *crr*, *ptsG* gene, or any combination thereof;
(ix) overexpression of endogenous *glf* gene or *glk* gene or heterologous expression of exogenous *glf* gene or *glk* gene;
(x) deletion, knockout, or knockdown of endogenous *pta*, *poxB*, *ldhA* gene, or any combination thereof;
(xi) overexpression of endogenous *ppsA, pyc, pck* gene or any combination thereof; or heterologous expression of exogenous *ppsA, pyc, pck* gene or any combination thereof;
(xii) any combination of (iii) to (xi).

27. The engineered microbial cell of any one of claims 1 to 26, comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); (ii) deletion, knockout, or knockdown of endogenous *gnd* gene and/or *zwf* gene; and (iii) deletion, knockout, or knockdown of endogenous *gltBD* gene;
optionally the engineered microbial cell further comprises any one of the following:
(iv) deletion, knockout, or knockdown of endogenous *tktA* gene and/or *tktB* gene;
(v) deletion, knockout, or knockdown of endogenous *talA* gene and/or *talB* gene;
(vi) modification or replacement of endogenous *gltA* gene to contain a rare codon;
(vii) deletion, knockout, or knockdown of endogenous *ppc* gene;
(viii) deletion, knockout, or knockdown of endogenous *ptsH*, *ptsI*, *crr*, *ptsG* gene, or any combination thereof;
(ix) overexpression of endogenous *glf* gene or *glk* gene or heterologous expression of exogenous *glf* gene or *glk* gene;
(x) deletion, knockout, or knockdown of endogenous *pta*, *poxB*, *ldhA* gene, or any combination thereof;
(xi) overexpression of endogenous *ppsA, pyc, pck* gene or any combination thereof; or heterologous expression of exogenous *ppsA, pyc, pck* gene or any combination thereof;
(xii) any combination of (iv) to (xi).

28. The engineered microbial cell of any one of claims 1 to 27, comprising:
(i) an exogenous transketolase possessing a ratio of Km for F6P to Km for X5P that is less than 1:1 (e.g., 1:4) and a ratio of Km for S7P to Km for X5P that is less than 1:1 (e.g., 1:4); (ii) deletion, knockout, or knockdown of endogenous *gnd* gene and/or *zwf* gene; (iii) deletion, knockout, or knockdown of endogenous *gltBD* gene; (iv) deletion, knockout, or knockdown of endogenous *tktA* gene and/or *tktB* gene; and (v) deletion, knockout, or knockdown of endogenous *talA* gene and/or *talB* gene;
optionally the engineered microbial cell further comprises any one of the following:
(vi) modification or replacement of endogenous *gltA* gene to contain a rare codon;
(vii) deletion, knockout, or knockdown of endogenous *ppc* gene;
(viii) deletion, knockout, or knockdown of endogenous *ptsH*, *ptsI*, *crr*, *ptsG* gene, or any combination thereof;
(ix) overexpression of endogenous *glf* gene or *glk* gene or heterologous expression of exogenous *glf* gene or *glk* gene;
(x) deletion, knockout, or knockdown of endogenous *pta*, *poxB*, *ldhA* gene, or any combination thereof;
(xi) overexpression of endogenous *ppsA, pyc, pck* gene or any combination thereof; or heterologous expression of exogenous *ppsA, pyc, pck* gene or any combination thereof;
(xii) any combination of (vi) to (xi).

29. The engineered microbial cell of claim 1, comprising:
(1) (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); and (ii) deletion, knockout, or knockdown of endogenous *gnd* gene;
(2) (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); (ii) deletion, knockout, or knockdown of endogenous *gnd* gene; and (iii) deletion, knockout, or knockdown of endogenous *talA* gene and *talB* gene;
(3) (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); (ii) deletion, knockout, or knockdown of endogenous *gnd* gene; and (iii) deletion, knockout, or knockdown of endogenous *gltBD* gene;
(4) (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); (ii) deletion, knockout, or knockdown of endogenous *gnd* gene; (iii) deletion, knockout, or knockdown of endogenous *gltBD* gene; (iv) deletion, knockout, or knockdown of endogenous *tktA* gene and *tktB* gene; (v) deletion, knockout, or knockdown of endogenous *talA* gene and *talB* gene; and (vi) overexpression of endogenous *ppsA* gene;
(5) (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); (ii) deletion, knockout, or knockdown of endogenous *gnd* gene; (iii) deletion, knockout, or knockdown of endogenous *gltBD* gene; (iv) deletion, knockout, or knockdown of endogenous *tktA* gene and *tktB* gene; (v) deletion, knockout, or knockdown of endogenous *talA* gene and *talB* gene; (vi) modification or replacement of endogenous *gltA* gene to contain a rare codon; and (vii) overexpression of endogenous *ppsA* gene; or
(6) (i) an exogenous transketolase possessing a ratio of Km for F6P to Km for X5P that is less than 1:1 (e.g., 1:4) and a ratio of Km for S7P to Km for X5P that is less than 1:1 (e.g., 1:4); (ii) deletion, knockout, or knockdown of endogenous *gnd* gene; (iii) deletion, knockout, or knockdown of endogenous *gltBD* gene; (iv) deletion, knockout, or knockdown of endogenous *tktA* gene and *tktB* gene; (v) deletion, knockout, or knockdown of endogenous *talA* gene and *talB* gene; and (vi) overexpression of endogenous *ppsA* gene.

30. The engineered microbial cell of claim 29, further comprises any one of the following:
(i) deletion, knockout, or knockdown of endogenous *ppc* gene;
(ii) deletion, knockout, or knockdown of endogenous *ptsH*, *ptsI*, *crr*, *ptsG* gene, or any combination thereof;
(iii) overexpression of endogenous *glf* gene or *glk* gene or heterologous expression of exogenous *glf* gene or *glk* gene;
(iv) deletion, knockout, or knockdown of endogenous *pta*, *poxB*, *ldhA* gene, or any combination thereof;
(v) overexpression of endogenous *pyc, pck* gene or any combination thereof, or heterologous expression of exogenous *pyc, pck* gene or any combination thereof;
(vi) any combination of (i) to (v).

31. The engineered microbial cell of any one of claims 1 to 30, which is bacteria, fungi, or yeast.

32. The engineered microbial cell of any one of claims 1 to 30, which is *Escherichia coli, Corynebacterium glutamicum*, *Bacillus subtilis*, or *Bacillus licheniformis*.

33. Use of the engineered microbial cell of any one of claims 1 to 32 in biosynthesis of aromatic amino acid.

34. A method for producing aromatic amino acid, comprising culturing the engineered microbial cell of any one of claims 1 to 32 in a medium.

35. The method of claim 34, further comprising recovering aromatic amino acid from the culture.

36. A method for decreasing the production of by-products and/or increasing the yield or conversion rate of aromatic amino acid in aromatic amino acid biosynthesis, comprising introducing a genetic modification as defined in any of claims 1-30.
